# EUROPEAN PATENT SPECIFICATION

(11) **EP 1 294 922 B1**
(45) Date of publication and mention of the grant of the patent: **14.04.2010**
(21) Application number: 01930891.5
(22) Date of filing: 27.04.2001
(51) Int. Cl.: A61K 31/41, A61K 31/427, A61K 31/17, A61K 33/24, A61P 35/00, A61L 27/18, A61L 27/54

(54) **TREATING CANCER USING ORGANELLE CRYSTALLIZING AGENTS**
BEHANDLUNG VON KREBS UNTER VERWENDUNG VON ORGANELLKRISTALLISIERENDEN VERBINDUNGEN
TRAITEMENT DU CANCER PAR UTILISATION D'AGENTS DE CRISTALLISATION D'ORGANITE

(30) Priority: 29.04.2000 CN 01101092; 15.09.2000 US 663559; 12.10.2000 US 687342; 13.11.2000 CN 00129316
(43) Date of publication of application: 26.03.2003
(73) Proprietor: Kong, Qingzhong, Jinan (CN)
(72) Inventor: Kong, Qingzhong, Jinan (CN)
(74) Representative: Evens, Paul Jonathan
(86) International application number: PCT/US2001/013730
(87) International publication number: WO 2001/082780

(56) References cited:
- US-A- 5 726 009
- CHAUHAN P S: "TETRAZOLIUM SALTS ADRENERGIC BLOCKADE BY BLUE TETRAZOLIUM" EXPERIENTIA (BASEL), vol. 5, no. 25, 1969, pages 511-512, XP009082203 ISSN: 0014-4754
- WANG H ET AL: "USE OF OXIDIZING DYES IN COMBINATION WITH 2-CYANOCINNAMIC ACID TO ENHANCE HYPERTHERMIC CYTOTOXICITY IN L929 CELLS" CANCER RESEARCH, AMERICAN ASSOCIATION FOR CANCER RESEARCH, BALTIMORE, MD, US, vol. 47, 1 July 1987 (1987-07-01), pages 3341-3343, XP008054948 ISSN: 0008-5472
- WEBER ET AL.: 'Histochemical and ultrastructural characterization of vacuoles and spherosomes as components of the lytic system in hyphae of the fungus botrytis cinerea' THE HISTOCHEM. J. vol. 31, May 1999, pages 293 - 301, XP002947605
- LIU ET AL.: 'Mechanism of cellular 3-(4,5-dimethylthiazol-2-yl)-2,5-diphenylte trazolium bromide (MTT) reduction' J. NEUROCHEM. vol. 69, no. 2, August 1997, pages 581 - 593, XP002947606
- ABE ET AL.: 'Both oxidative stress-dependent and independent effects of amyloid beta protein are detected by 3-(4,5-dimethylthiazol-2-yl)-2,5-diphenylte trazolium bromide (MTT) reduction assay' BRAIN RES. vol. 830, May 1999, pages 146 - 154, XP002947607
- VERMA ET AL.: 'Gene therapy-promises, problems and prospects' NATURE vol. 389, September 1997, pages 239 - 242, XP002947608

## Description

### FIELD OF THE INVENTION

The present invention provides cellular organelle crystallizing agents (e.g. tetrazolium violet) for treatment of cancer.

### BACKGROUND OF THE INVENTION

While space scientists are planning to land on the planets that are much farther from us than the Moon and Mars, biomedical researchers are still waging in the battle of combating cancer-a tiny cell. Are the former much more clever than the latter? Biomedical scientists have succeeded in sequencing almost of the human genome and in copying some species of mammals and insects, things we dared not imagine a few decades ago. What is the real problem in biological studies, such as cancer research?

As far as space scientists are concerned, the more calculations they make, the less possibility they will miss their targets. In rocketing astronauts off into high space, processes involve physical and/or chemical principles, always of nature, which have been known and could be predicted using the fundamentals and principles of mathematics. In the biological studies, the case is different. What we are facing and dealing with are viable cells, particularly the aggressive cancer cells that might have millions of proteins and genes which may regulate each other, compensate each other, or modify each other. Therefore, some aspects of our thinking concerning cancer prevention and treatment are not only inconsistent, but are often even contradictory (Kong, et al., (2000) Med Hypotheses 55(1): 29-35). The dual effect concept and self-defense mechanism (Kong, Q. et al., (1998) Med Hypotheses 51: 405-409) plus the threshold theory (Kong, Q., et al., (2000) *supra*) might have shed light on the paradoxical observations and have explained at least partially the origin of these paradoxical findings. However, the primary robust reason for the dual effect and self-defense responsible for the threshold is unclear and is expected to be better understood in terms of a cell brain theory.

### LIFE ORIGINS TEACH THE CELL BRAIN CONCEPT.

Unexceptional to the universe, the origin of life has long been a most unsolvable riddle to evolutionists. According to molecular phylogenetic analyses performed lately, plants, animals, and fungi all are claimed to be diverged from a common ancestor during a short period of time, ∼1.5 billion years ago (Riechmann, J.L., et al., (2001) Science 290: 2105-10), for a set of particular transcription factor families and genes are found to be specific to each lineage, and some present in all four tested organisms (Riechmann, J.L., et al., *supra*). Similarly, the plant-specific GRAS proteins were thought to be distant relatives of the animal-specific STAT, based on a similar arrangement of related functional domains (Richards, D.E. et al., (2000) Bioessays 22: 573-577), and the trihelix DNA-binding domain, present in plants (Nagano Y (2000) Plant Physiol 124; 491-4), might have evolved from the MYB domain, found in all eukaryotes (Riechmann, J.L., et al., *supra*). Attempts to explain the genotypic or phenotypic similarities between different plants, animals, humans, and/or microorganisms are more than a few (Vision, T.J. et al., (2000) Science 290: 2114- 2116). To accept such explanations, one would have to believe that human and other animals have a common ancestor for they all have brain, ears, one heart, and two eyes. One would also argue that all airplanes and ground transportation vehicles have evolved from a common ancestor for they have shared many identical parts, or they all have evolved from an engine for they all have engine. Can one really dispense with the need for a creator to originate life or equipment either now or in the beginning? Needless to go back to billion years ago, just comparing the eyes of a human, the highest animal, with that of a bird, how could one know who originated from who, for a bird has sharper eyes and a human has a more complicated brain with its creativeness? How could one explain, at the point of evolution, the relation between an animal and the digestive tract parasitic bacteria that provide a group of vitamins necessary to the host? And how could one image the way under which eyes or hearing system functioned when they were half evolved? The argument about life origins may be lasting forever if people don't believe the purpose and importance of life, for people will never be able to go back a billion years (or even a second) ago, and the whole process through which life was created was finished and won't be repeated. Just like being in a labyrinth, if one doesn't believe the existence of the gate, how could he find the exit? Nevertheless, the existence of the similarities or homologs between the low ranked microorganisms and highest mammalians at the molecular, cellular, or even physiological levels is unmistakable, no matter what we believe, and is to be greatly appreciated for they have provided general principles for humans to follow.

Because such similarities or homologs between human and animals or microorganisms exist, there is the opportunity and necessity to do pre-clinical experiments in medical and biological fields. Indeed, much of our current understanding of molecular mechanisms controlling mammalian cell cycle and transcriptional process originates with studies of bacterium, fungi or other organisms (Kirchaier, A.L. and Rine, J. (2001) Science 291: 646-650; Laub, M.T. et al., (2000) Science 290: 2144- 2148; Li, Y.C. et al., (2001) Science 291: 650; Riechmann, J.L., et al., *supra;* Berset, T. et al., (2001) Science 291: 1055-1058). The thought on the mechanism that controls the exit of cytokinesis in vertebrate cells (Piel, M. et al., (2001) Science 291: 1550-1553), for example, come out of the understanding of the checkpoint mechanism seen in yeast cells (Bloecher, A. et al., (2000) Nature Cell Biol 2: 556-558). In the pharmacodynanics, almost all data about the drugs used in human patients came from animals at varying levels. Studies on the low level microorganisms or animals have long been used and have provided enormous, invaluable information or insights for better understanding of the human cell biology, such as cell cycle control, signal transduction, and human diseases. Although the complexity in protein domain organization increases with the complexity of the organism (Rubin, G.M. et al., (2000) Science 287: 2204-2215), the known and yet-to-be-known similarities among different microorganisms and mammals do not prevent the inventor from raising the question, "why don't we borrow our available knowledge, particularly that on the systems of animals and humans, to help better understanding some unsolved events in cells - the basic constitutive unit of the human body?"

Using the women's menstrual cycle as an example, if one just focused on the changes seen in the ovary and/or uterus, he could not know the mechanism that regulates the local periodical changes in both the hormones and histology? It is the same as a mammal with a growing tumor on the spinal cord or brain. If one is lacking in knowledge on the anatomy and function of nervous system, how could he explain why the patient animal lost their motility at the normal extremities. The answer is very clear to today's scientists, but would not have been so a century ago. Similarly, in the face of thousands of transcriptional factors (Laub, M.T. et al., *supra*) and thousands of genes, how could one obtain a clear outlook of the mechanisms through which cell cycle and other complicated processes are regulated? Couldn't we believe the existence of a powerful coordinator in a microorganism or mammal cell? As with most cases, otherwise, researchers will inevitably become the characters of a tale "blind men sizing up an elephant". For the purpose of clarity, the coordinator is referred to herein as "cell brain", being reminiscent of the long known "brain" in an animal or a human.

### LATEST STUDIES SUPPORT THE CELL BRAIN HYPOTHESIS

All non-surgical anticancer therapies such as radiotherapy, chemotherapy, photodynamic therapy, immunotherapy, electric/chemotherapy, hyperthermia therapy, hyperbaric oxygen therapy, and ischemia/reperfusion therapy, have long been found to be effective for cancer therapy. However, all has been limited by the development of resistance including both natural and acquired (Kong, Q. et al (1998) supra, (2000) *supra*). The cells, both normal and transformed, have their own natural or acquired defense mechanisms, which are called self-defense and maintain the balance of intracellular reactive oxygen species and antioxidants. Throughout the life span of both normal and transformed cells this balance is continually in flux. Imbalance in the antioxidant system can lead to the inability of a cell to cope with exogenous peroxidative attacks. These alterations might then be correlated with the malignant transformation of the cell or cell disfunction leading to disease or death (Kong, Q., et al., (1998) *supra*). Actually, this self-defense is just the intrinsic nature of human being and other creatures, which constitute a prerequisite ensuring them to be used to the changing environment. As a human, one must be clear what the self-defense is about and how it works. One can cell the harmful and dangerous things, therefore, he will behave protectively. As far as the treated cells (such as cancer cells or microorganisms) are concerned, similar mechanisms might also be involved, to which researchers have not paid enough attention.

For a given treatment, the outcome is affected and determined by many factors, including both the cells themselves and the treatments exposed (see, e.g. Kong, Q., et al *supra*). Briefly, in normal cells, low levels of non-surgical treatments (e.g. reactive oxygen species (ROS) production) may cause the adaptive production of antioxidants (Melov, S. et al., (2000) Science 289: 1567-9). If the antioxidant activity maintains in equilibrium with the treatment, a balance between oxidation and anti-oxidation within the cell is maintained. If the cell is then exposed to more ROS, the adaptive capacity of the antioxidants is overwhelmed and cellular DNA damage, followed by neoplastic transformation may be initiated. As an alternative, however, the presence of an overwhelming amount of ROS may in turn exhaust the available antioxidant activity, triggering apoptosis. For the established tumors, low levels of ROS production may stimulate tumor growth, or induce the adaptive production of antioxidants, which may contribute to the development of acquired drug resistance or other resistances depending on specific situations. As the level of ROS within the cell increases, the ratio of ROS to antioxidant reaches a point where the destructive capacity of the ROS and the protective effect of the antioxidants are balanced. Beyond this level, however, further increases in ROS or reductions in antioxidant activity will result in an imbalance in the tumor cell antioxidant system leading to cell death or an increased sensitivity of the tumor cell to other cytotoxic treatments.

Based on these findings, the inventor of this invention can not refuse to acknowledge the notion that there is cellular organelle(s) responsible for integrated regulation of a cell life. Could this organelle be the nucleus or other non-nucleus organelle?

### COMPARATIVE STUDIES LINK THE CENTROSOME TO THE CELL BRAIN

Based on the findings described above plus the conventional understanding of the regulation to cell cycles and gene expressions, one must raise a question: what comes first in these alterations, proteins or genes? Although this universal feature was appreciated long ago, our understanding of how robustness is attained at the cellular and molecular level remains quite limited. Still, like the question about chickens and eggs or the question regarding life origins as mentioned above, there will be no answers to satisfy all the people.

The regulation of gene expression at the level of transcription influences or controls many of the biological processes in a cell or organism, such as progression through the cell cycle, metabolic and physiological balance, and responses to the environment (Riechmann, J.L., et al., *supra*). This may be the reason for phenotypic stability in living systems in the face of a great variety of perturbations arising from environmental changes, stochastic events, and genetic variation (Hartman, J.L. et al., (2001) Science 291: 1001-1004). Development is also based on the cellular capacity for differential gene expression and is often controlled by transcription factors acting as switches of cascades (Scott, M.P. (2000) Cell 100: 27-40). Gene regression and activation play pivotal roles in the differentiation of totipotent cells into different cell types, each of which selectively and stably expresses only a subset of the genes in the genome (Kirchaier, A.L. and Rine, J. *supra*). The alterations in the expression of genes coding for transcriptional regulators are therefore emerging as a major source of the diversity (Carroll, S.B. (2000) Cell 101: 577-580). Therefore, the robustness responsible for the development of the "self defense" (Kong, Q. et al., (1998) *supra*) and the "threshold" (Kong, Q. et al., (2000) *supra*) seems to be less related to nucleus than it was thought to be.

DNA replication can play a role in changing patterns of gene expression (Dalgaad, J.Z. and Klar, A.J. S. (1999) Nature 400: 181-184). Under physiological conditions, however, DNA is up to 100,000-fold more stable than RNA (Li, Y. and Breaker, R.R. (1999) J Am Chem Soc 121: 5364-5372), and 100-fold more resistant to hydrolytic degradation than proteins (Smith, R.M. and Hansen, D.E. (1998) J Am Chem Soc 120: 8910-8913). This stability, coupled with the complementary character of nucleotides, makes DNA an ideal molecule for information storage and transfer (Breaker, R.R. (2000) Science 290: 2095-2096). The changes in gene expression and differentiation are reported to occur independently of DNA replication (Kirchaier, A.L. and Rine, J. *supra*; Li, Y.C. et al., *supra*). Silencing of large chromosomal domains involves specialized, heritable chromatin structures that repress transcription in a gene-independent fashion (Li, Y.C. et al., *supra*). By measuring changes in the level of DNA supercoiling, the authors (Li, Y.C. et al., *supra*) demonstrated that establishment of silencing in a nonreplicating template is accompanied by alterations in chromatin structure, and is Sir-dependent (Li, Y.C. et al., *supra*). Although lots of events could control the establishment of silencing through the generation of specific cell cycle signals or changes in nuclear structures, what was once considered the most likely event
- replication of the DNA prior to silencing - does not appear to be among them (Smith, J.S. and Boeke, J.D. (2001) Science 291: 608-09). Putting together the results that establishment of silencing involves something other than DNA replication during passage between early S and M phase (Li, Y.C. et al., *supra*), that establishment of silencing is Sir-dependent, and that activity of a critical silencing component (such as a Sir protein or silencer binding protein) is regulated in a cell cycle-dependent manner (Li, Y.C. et al., *supra*), the inventor of this invention strongly believes that the complex comprising the centrosome, centrioles and the connecting filaments, instead of nucleus, best fits the cell brain of this invention.

The centrosome, a central body (or the major microtubule organizing center (MTOC) of the cell, is composed of two centrioles surrounded by the so-called pericentriolar material (PCM) or centrosome matrix, which consists of a complex thin filament network and two sets of appendages (Paintrand, M. et al., (1992) J Struct Biol 108: 107-128). The best known function of *the* centrosome is the nucleation of microtubules and the formation of bipolar spindles, which form the cellular railroad and direct events during mitosis (e.g. the equal separation of chromosomes) and control the organization, temporal and spatial distribution of cell structures and movement during interphase (e.g. cargoes, vesicles, and endoplasmic reticulum) (Hsu, L.C. and White, R.L. (1998) Proc Natl Acad Sci U S A 27; 95(22): 12983-8; Tanaka, T., et al., (1999) Cancer Res 59(9): 2041-4). Texturally, the centrosome, a thixotropic inconspicuous blob, seems to be made of the materials similar to that of the brain of other organisms. The centriole-embedded PCM or centrosome matrix seems to be reminiscent of the white matter-embedded gray matter of a mammal brain. The arrangement of the two centrioles as nine triplets of microtubules (Paintrand, M. et al., supra) appears to be reminiscent of the arrangement of the two hemispheres of the animal brain and craniocerebral nerves, with the lost three pairs seen in the "cell brain" possibly being those that are associated with the visioning, hearing and talking in the brain of mammals. The centriole cylinder is made of two parts, a proximal one is more like the classical organization of the basal body with nine opened triplets and a distal one which is more reminiscent of the organization of an axoneme with nine doublets oriented in a more tangenitial manner (Paintrand, M. et al., supra). It is at this end (not necessarily the same number) that, in certain cell types, primary cilium, or flagellar filaments (Yonekura, K. et al., (2000) Science 290: 2148-2152) or cytoskeletons would grow (Hsu, L.C. and White, R.L. supra; Tanaka, T., et al., supra). As the unique function to the mammal brain, the activity of memory in the cell brain could not be excluded, and it, if any, might be an unsolvable riddle for human beings.

Malignant tumors generally display abnormal centrosome profiles, characterized by an increase in size and number of centrosomes, by their irregular distribution, abnormal structure, aberrant protein phosphorylation, and by increased microtubule nucleating capacity in comparison to centrosomes of normal tissues (Lingle, W.L. et al., (1998) Proc Natl Acad Sci USA 95(6): 2950-5; Doxsey, S. (1998) Nat Genet 20(2):104-6; Sato, N. et al., (1999) Clinical Cancer Res 5: 963-70). Among the abnormalities, centrosome hyperamplification is found to be more frequent in a variety of tumor types (Hinchcliffe, E.H., et al., (1999) Science 283(5403):851-4; Weber, R.G., et al., (1998) Cytogenet Cell Genet 83:266-269). Although the precise mechanisms by which the centrosomes are (up) regulated during cell cycle are largely unknown, the overexpression of centrosomal kinases or the lack of tumor suppressor genes are observed universally in malignant tumors (Zhou, H., et al., (1998) Nat Genet 20(2): 189-93).

In summary, the different organs (or systems) of the body are built from different populations of cells, and the different parts of the cell are built from the different organelles, a general principle in biological structure through which a function is assigned to the different organs (or systems) of the body or the different parts of the cell. To a cell, particularly that which divides by mitosis, the organelle equivalent of the animal brain is most likely to be the complex comprising the centrosome, centrioles and the connecting filaments. In other words, this complex best represents the cell brain both structuraly and functionally. The nucleus of the cell probably acts mainly as a secure storage of DNA, an ideal copying machine or a molecule for information storage. When and what products are needed is not determined by the copier itself.

Except for the known key proteins (such as SKP1p, cyclin-dependent kinase 2-cyclin E (Cdk2-E), kendrin, protein kinase C-theta, and EB1 protein), a variety of new cell cycle-regulated kinases or tumor suppressor genes are found to be located in or to be core components of the centrosome. They include Nek2, protein kinase A type II isozymes, heat shock Cognate 70 (HSC70), PH33, AIKs, human SCF(SKP2) subunit p19(SKP1), STK15/BTAK, C-Nap1, Tau-like proteins, cyclin E, retinoblastoma protein pRB and BRCA1. These proteins are required in the initiation of DNA replication and mitotic progression (Gstaiger, M., et al., (1999) Exp Cell Res 15;247(2):554-62).

As with most biological processes and particularly with the processes of cell cycle control and signal transduction, the story is more complicated than appears at first sight. It is likely that the proteins or kinases identified to be associated with centrosome dysfunction are only a couple of many in a complex pathway (or parallel pathway) that controls centrosome assembly and function. Support for this idea comes from other new molecules that were reported lately. For example, BTAK/AIK1 (Tanaka, T., et al., *supra*), AIK3 (Kimura, M., et al., (1999) J Biol Chem 274(11):7334-40), Mdm2 (Carroll, P.E. et al., (1999) Oncogene 18;18(11):1935-44) and STK15/BTAK (Zhou, H., et al., *supra*) are also associated with centrosome dysfunction. The changes such as the loss of p53 tumor suppressor protein and/or the overexpression of these centrosome kinases may cause abnormal centrosome function, abnormal spindle formation, and chromosome segregation (Carroll, P.E. et al., *supra;* Zhou, H., et al., *supra;* Tanaka, T., et al., supra).

It is therefore not difficult to envision how hard could it be to control the aggressive division of cancer cells by inhibiting one or group of molecules. There are no methods or agents that have been reported to choose the centrosome, centrioles and the connecting filaments as a target for this purpose.

In the past decades, almost all anti-cancer techniques have focused on the inhibition of the elevated cancer products or the oncogenes that code these products. As the powerful regulating ability and self-defense mechanisms of the aggressively growing cancer cells (Kong, Q. et al (1998) *supra*), which the inventor believe originated from the cell brain coordination, is greatly ignored, a satisfying outcome following traditional therapies has never been obtained. The commonly recognized reason is likely the development of a variety of resistances prior to recurrence. Similarly, the integrated regulation is greatly ignored in the studies of cell biological processes and molecular progresses, which might be the reason that caused the inconsistent or even contradictory explanations of findings (Kong, et al., (2000) *supra*). Thus, there is a need for the better and integrated understanding of cell growth, aging and their regulation to facilitate the development of novel, more effective cancer therapies.

In addition, most available methods for the visualization of the centrosome and other important cellular organelles and/or cytoskeleton are based on the antigen-antibody reaction (Lingle, W.L. et al., *supra;* Brinkley, B.R., et al., (1998) Cell Motil Cytoskeleton 41(4): 281-8; Doxsey, S. *supra;* Carroll, P.E., et al., *supra;* Hinchcliffe, E.H., et al., *supra;* Xu, X., et al., (1999) Mol Cell 3(3): 389-95). These techniques have been proved to be very costly, poorly reproducible, time consuming, and requiring of very strict conditions. Particularly, these methods can not be used to demonstrate the dynamic states of cells.

It is against this background, this disclosure provides additional experimental evidence supporting the cell brain theory. Based on the theory, this disclosure treats the centrosome and centrioles as a priority target for cancer therapy and for understanding of other cell procedures.

An α-adrenergic receptor blocking activity of blue tetrazolium is disclosed in:
CHAUHAN P S: "TETRAZOLIUM SALTS ADRENERGIC BLOCKADE BY BLUE TETRAZOLIUM" EXPERIENDA (BASEL), vol. 5, no. 25,1969, pages 511-512

The article:
WANG H ET AL: "USE OF OXIDIZING DYES IN COMBINATION WITH 2-CYANOCINNAMIC ACID TO ENHANCE HYPERTHERMIC CYTOTOXICITY IN L929 CELLS" CANCER RESEARCH, AMERICAN ASSOCIATION FOR CANCER RESEARCH, BALTIMORE, MD, US, vol. 47, 1 July 1987 (1987-07-01), pages 3341-3343 discloses that two widely used oxidixing dyes, 2,3,5-triphenyltetrazolium chloride and methylene blue, can greatly potentiate hyperthermic cytotoxicity when administered simultaneously with 2-cyanocinnamic acid. The same compounds are virtually non-toxic to L929 cells if administered alone at 42°C.

### SUMMARY OF THE INVENTION

The present invention provides a tetrazolium salt for use in the treatment of cancer as defined in claim 1. This treatment is expected to freeze the organelles, particularly the centrosome and centrioles as a whole, instead of just inhibiting one or group of molecules. The crystals formed in the cellular organelles are visible. Therefore, this disclosure also provides a biochemical method for visualizing cellular organelles and/or cytoskeletons, by treating tissues or cells with crystallizing agents. The crystallizing agents or compounds used in this invention are a variety of tetrazolium salts. The cell-mediated reduction of some tetrazolium salts has long been used as a cell number-counting method (Berridge, M.V. and Tan, A.S. (1993) Arch Biochem Biophys 303(2): 474-482; Bernas, T., et al., (1999) Biochim Biophys Acta 12;1451(1):73-81; Abe, K., and Saito, H., (1999) Brain Res 29;830(1):146-54; Liu, Y., et al., (1997). J Neurochem 69(2):581-93; Abe, K., and Saito, H., (1998) Neuroscience Res 31: 295-305). In the visualization of cellular organelles and/or cytoskeletons, or in the treatment of cancer, the application of tetrazolium salts has never been mentioned.

The inventor of this invention has found that tetrazolium salts can specifically concentrate on cellular organelles and/or cytoskeletons of a variety of cells and tissues, with the formation of visible crystals catalyzed by the enzymes in these places. The crystallization of the centrosome irreversibly blocks the cells in S phase and M phase. It also interferes with the apoptosis induced by apoptosis inducing agents or treatments. Therefore, it adds additional evidence suggesting that the complex comprising of the centrosome, centrioles and the connecting filaments in the centrosome matrix best or exclusively represent the cell brain. The formation of the visible crystals in cellular organelles and/or cytoskeletons through a biochemical approach instead of the complicated immune methods provides a less-costly, very simple, quick, and effective method for the visualization of cellular organelles and/or cytoskeleton. Crystallization may interfere with the signal transduction from and/or to a targeted organelle, such as centrosome, in cancerous cells instead of normal cells. Therefore, this disclosure provides a potential tool for a better integrated understanding of other cellular processes such as cell cycle regulation, signal transduction, development biology, antigen editing, receptor regulation, aging, gene expression, transcription regulation, carcinogenesis, cell motility, apoptosis regulation and intercellular activities. Additionally, the formation of crystals in the organelles is helpful to the isolation of said organelles, and provides a method for isolation of the organelles and proteins thereof, and a method for distinguishing cancer cells from the normal, and a method for screening inhibitors of the said enzymes.

There is a desire to provide a novel theory for integrated understanding of cellular processes, which guides the development of a novel method for treating cancer (or for cell biology study).

There is also a desire to provide a novel class of agents having improved chemotherapeutic efficacy (or having cellular organelle visualizing ability).

There is also a desire to provide a novel method for cancer therapy (or for cell biology study) using cellular organelle and/or cytoskeleton crystallizing agents.

There is also a desire to provide a method for selectively increasing the susceptibility of tumor cells to other non-surgical anticancer therapies (or a method for selectively inhibiting one or more steps of a cell cycle).

There is also a desire to provide a method for treating cancer that has minimal side effects (or a method that selectively visualizing cancerous cells).

There is also a desire to provide a method for treating cancer that reduces or eliminates the potential for the development of tumor resistance (or a method selectively inhibiting the centrosome).

There is also a desire to provide a composition for treating cancer comprising a therapeutically effective amount of a crystallizing agent.

There is also a desire to provide a composition for treating cancer comprising therapeutically effective amounts of a crystallizing agent and a therapeutic agent

Additional objects, advantages and novel features of this invention shall be set forth in part in the description that follows, and in part will become apparent to those skilled in the art upon examination of the following specification or may be learned by the practice of the invention. The objects and advantages of the invention may be realized and attained by means of the compounds particularly pointed out in the appended claims.

To achieve the foregoing and other objects and in accordance with the purposes of the present invention, as embodied and broadly described therein, the present invention is defined in appendant independent claim 1 to which reference should now be made. Embodiments of the invention are defined in the appendant dependent claims to which reference should also now be made.

### BRIEF DESCRIPTION OF THE DRAWINGS

The accompanying drawings, which are incorporated in and form a part of the specification, illustrate the preferred embodiments of the present invention, and together with the descriptions, serve to explain the principles of the invention.
Figures 1A and 1B show the dose-dependent cell killing effect of tetrazolium red (TR) (Figure 1A) and tetrazolium violet (TV) (Figure 1B) on 9L gliosarcoma tumor cells. The concentration of TR and TV was same (mg/ml), as indicated in the figures: 0, 5, 25, 50, and 250.
Figure 2 shows the synergistic effect of crystallizing agent and chemotherapeutic agents on 9L gliosarcoma.
Figure 3 shows the reduction in tumor size of established 9L gliosarcoma in rats following treatment with tetrazolium salts impregnated into biodegradable polymer.
Figure 4 shows the crystallization of centrosomes of SHP77 (SCLC) cells following treatment with tetrazolium violet (TV).
Figure 5 shows the crystallization of microtubules of 9L gliosarcoma cancer cells following treatment with MTT.
Figure 6 shows the crystallization of mitochondrion (or lysosomes) of 9L gliosarcoma cells following treatment with MTT.
Figure 7 shows the crystallization of nuclear envelopes (or endoplasmic reticulums or Golgi's complex) of 9L gliosarcoma cells following treatment with tetrazolium red (TR).

### DETAILED DESCRIPTION OF THE PREFERRED EMBODIMENTS

The inventor of the present invention has found that dehydrogenases such as succinate dehydrogenase (SDH) and/or other enzymes such as oxidation reductases are highly expressed in cancer cells in contrast to the normal originating partners. Except in mitochondrion, it is widely expressed in other cellular structures such as, but not limited to, centrosomes, centrioles, microtubules, flagella, and nuclear envelopes etc. This finding *is contrary to* the established view on the cellular distribution of SDH in mammalian cells. When tetrazolium salts are present with viable cells, cellular dehydrogenases such as SDH, which is usually anchored on the cellular structures, can reduce the tetrazolium salts into related visible crystals that can be visualized under proper conditions. The formation of crystals may results in both mechanical injuries to cellular structures that are usually very flexible and functional impairment. For example, crystallization of microtubules not only damages cell membranes but also changes the moving track of small molecules and signal transduction, or alters the check-point mechanism during cell mitosis. Crystallization of centrosomes not only limits the formation of spindles and the segregation of chromosomes but also interferes with the signal transduction from and/or to the centrosome. Crystallization of the centrosome and centrioles, the said cell brain of the present invention, irreversibly blocked cells in both S phase and M phase. In other words, all key enzymes located in the said cell brain and other targeted structures of the cell could not function normally. The formation of crystals may act as a restraint to the separation and movement of the duplicable organelles such as the said cell brain and cytoskeletons. The cellular structures that are rich in SDH and other key enzymes in these structures will be functionally and structurally frozen or locket. In addition, the cell death induced by the crystallizing agents is different from the known forms of death (apoptosis and necrosis). For the purpose of clarity, this "cellular-organelle-crystallization-induced-death" is referred to herein as "COCID". The more the enzymes are expressed, the more the crystals are formed.

Based on these findings, the inventor of this invention takes full advantage of the cell brain theory, and uses the oncogene products, instead of inhibiting them, which is expected to avoid the critical problem of the development of resistances in cancer therapy. Compounds of this invention exert a cancer-killing effect through freezing the whole cellular organelle and/or cytoskeleton instead of inhibiting specific molecules. For example, the crystallization of a tumor cell's centrosome, the said cell brain, is like *freezing or locking* the "brain" of a mammal animal. However, the simple inhibition of a certain molecule as seen with the conventional methods is like *cutting off* a finger or at most one extremity of it or a leaf or a branch of a tree. Therefore, *the potential of the present invention as an alternative in the battle of conquering cancer is great*. The crystallizing agents may also be used for treating a patient with bacteria, fungus, virus, or parasite infection.

Based on the discovery, the present disclosure also features a method for visualizing cellular organelles and/or cytoskeletons, in tissue or in cell suspension, by treating tissue or cells with one or more crystallizing agents. The method includes obtaining a tissue sample or a fluid sample containing multiple cells from a variety of sources, such as, but not limited to mammalian, microorganisms, and cancers; then fixing the tissue sample; contacting the tissue sample or cell sample with a crystallizing agent under conditions that permit the formation of visible crystals through the reduction of said crystallizing agent by the enzymes located in said cellular organelles and/or cytoskeletons in said cells or tissues; and then visualizing cellular organelles and/or cytoskeletons having crystals formed on them. The method allows for easy and quick visualization of the structures of cellular organelles and/or cytoskeletons that are usually observed through complex immunofluorescence staining or under electronic microscope.

Due to the high expression of the dehydrogenases in cancer cells, this method is particularly useful in detecting the changes of the number and shapes of the neoplasm in their cellular organelles and/or cytoskeletons, thereby detecting neoplastic disease in the tissue or differentiating neoplastic cells from normal cells.

Since the dehydrogenating reaction usually takes place in the viable cells, this method is particularly useful in monitoring the changes of the number and shapes of the cellular organelles and/or cytoskeletons of the cells at different time points. Therefore, this method is helpful for better understanding of the regulating mechanisms in cell cycle and signal transduction, particularly for screening and detecting the drugs that specifically target cellular organelles and/or cytoskeletons of the cells for their actions. Examples of such drugs are dehydrogenase inhibitors such as succinate dehydrogenase inhibitors.

The visualization of cellular organelles and/or cytoskeletons using the method of present biochemical approach instead of the complicated immune methods provides a less-costly, very simple, quick, and effective method for the visualization of cellular organelles and/or cytoskeleton of a tissue or cells. Said tissue or cell may be selected from the group consisting of normal mammalian tissue or cell, pathologic mammalian tissue or cell, and neoplastic tissue or cell, bacteria, parasite, fungus, and plants. The neoplastic tissue (cancer) or cell may be selected from the group consisting of carcinoma, sarcoma, carcinosarcoma, lymphoma.

Crystallization may interfere with the signal transduction from and/or to a targeted organelle, such as centrosome, the said cell brain. This disclosure also provides a potential tool for a better-integrated understanding of other cellular processes such as cell cycle regulation, signal transduction, development biology, antigen editing, receptor regulation, aging, gene expression, transcription regulation, carcinogenesis, cell motility, cell memory, apoptosis regulation, molecule transportation, cancer biology, degenerative disease, autoimmune disease, intracellular action, and intercellular activities.

Crystallization of cellular organelles may change the precipitating rate of the said organelles when they are spun, therefore, said Crystallizing agents may be used to isolate said organelles such as the centrosome and microtubules. Because the available procedure for the isolation of cellular organelles such as a centrosome usually changes the structures of the isolated organelles, prior crystallization before isolation would more expected, which will be helpful in preserving the spatial structure of the organelles. The isolated cellular organelles are then used for structural studies or to provide the organelle related proteins. Therefore, a protein bank for the organelle may be established. A preferred protein bank is for said cell brain consisting of centrosome and centrioles. The isolated and purified proteins may be used as antigen to produce antibody. They may be used for other purposes including but limited to structural study. The procedures of the isolation and purification of the said organelles and the organelle related proteins as well as the application of the proteins are known to the people in the art. The preferred crystallizing agents used for such purpose includes, but not limited to, tetrazolium violet, MTT, Tetrazolium blue, and the derivatives or analogues thereof. When performing such studies, the said cell brain, mainly "centrosome and centrioles", is preferably involved. The more preferred tetrazolium salts are tetrazolium violet and analogues or derivatives thereof.

In summary, this invention provides a tetrazolium salt for use in treating cancer, said salt being a cellular organelle crystallizing agent (COCA). This disclosure also provides a method for the visualization of cellular organelles and/or cytoskeletons of tissue or cells, a method for studying other cellular processes such as cell cycle regulation, signal transduction, aging, gene expression, transcription regulation, carcinogenesis, cell motility, apoptosis regulation and intercellular activities. This disclosure also provides a method for isolation of cellular organelles, a method for isolation and purification of proteins in said organelles.

The method of this treatment involves administering to a mammalian host, preferably a human host, a therapeutically effective amount of one or more crystallizing agents. The crystallizing agent is a compound or composition that *is* a substrate of cellular enzymes. The crystallizing agent is a tetrazolium salt such as tetrazolium blue, tetrazoilum violet or tetrazolium red, and the enzyme is a dehydrogenase such as succinate dehydrogenase (SDH). The crystallizing agent such as tetrazolium blue, tetrazoilum violet or tetrazolium red is catalyzed by said enzyme such as SDH with the formation of crystals. The formation of crystals may cause in the targeted cells a variety of changes, which include, but are not limited to, mechanical injury of cellular structures; deactivation of key enzymes; freezing of the targeted structures, blocking of signal transduction, cessation of cell mitosis and DNA replication. There is also disclosed a method of enhancing the effect of an anticancer therapy, which method comprises administering conjointly with the therapy an effective amount of a crystallizing agent. This disclosure also provides a composition suitable for administration to mammalian hosts comprising a therapeutically effective amount of a crystallizing agent. This disclosure also provides a composition suitable for administration to mammalian hosts comprising a mixture, in therapeutically effective amounts, of at least one crystallizing agent and at least one pharmaceutically acceptable agent.

The crystallizing agents are materials that can be reduced by cellular enzymes located in the organelles or cytoskeletons, with the formation of visible crystals. Cellular enzymes, mainly SDH, which is usually anchored on the cellular structures, will reduce the tetrazolium salts into related crystals that can be visualized under proper conditions. The visible crystals are in where the said enzymes are located, therefore providing a method for viewing the enzyme-hosting cellular structures or components. In addition, the inventor has also found that tetrazolium compounds have differential targeting structures. For example, TV forms crystals in centrosome, therefore specifically crystallizing centrosomes; MTT forms crystals in microtubules, mitochondrion, and nuclear envelopes or endoplasmic reticulum; and TR forms crystals in envelopes and/or other structures close to envelopes such as endoplasmic reticulum. Therefore different tetrazolium salts or analogues may specifically crystallize certain cellular organelles or sub-organelles. It also suggested that SDH may have different forms or subclasses which are located throughout cells.

The tissue sample or cell sample can be from any source, such as, but is not limited to mammalian, microorganism, plant, or parasite. The tissue sample or cell sample may be normal or neoplastic, from any part of a mammalian e.g., brain, neck, breast, lung, esophagus, liver, stomach, kidney, colon, rectum, skin, connective tissue, lymph node, blood vessel, nerve, ovary, bladder, uterus, testis, and bone. The tissue sample can be fresh, e.g., a biopsy sample, or can be from an archived sample, e.g., a frozen sample or a sample embedded in paraffin. The cell sample can from fluid, tissue cavity, tissue homogenate, tissue lavage, biopsy, cell lines, bone marrow and blood. The cell sample can be fresh, e.g., blood or bone marrow sample, or can be from an archived sample, e.g., a frozen cell lines.

Cellular organelles and cytoskeletons can have different appearances, dependent on sample origins, concentration of the agents used, and the duration of the contacting time.

Samples can be processed using methods known in the art, which includes tissue isolation and dissociation to release individual cells. Cells can be spun onto coverslips ("cytospun"), treated, and subjected to visualization under microscope. Cells are preferably visualized directly under microscope in culturing flasks.

Any tetrazolium compound, not limited to those described above such as TR, MTT, and TV, that can be reduced by cellular enzymes with the formation of visible crystals can be used in the invention.

Crystals formed by the reduction of said crystallizing agents are detected using the methods known in the art, e.g., microscope. Naked eyes can also tell the occurrence of the reaction by examining the change of the color in the culture medium. The method for visualizing cellular structures may be used with other techniques such as immunofluorescence, immunoperoxidase staining, flow cytometry, or Western blot hybridization.

The present disclosure is also directed to a kit containing one or more crystallizing agents, such as tetrazolium red, MTT, and tetrazolium violet. The kit may comprise a pharmaceutically acceptable carrier medium.

Additional objects and advantages of this invention shall be set forth in part in the description that follows, and in part will become apparent to those skilled in the art upon examination of the following specification or may be learned by the practice of the invention. The objects and advantages of the invention may be realized and attained by means of the tetrazolium salt defined in the appended claims.

Unless defined otherwise, all technical and scientific terms used herein have the same meaning as commonly understood by one of ordinary skill in the art to which this invention pertains. Although any methods and materials similar or equivalent to those described herein can be used in the practice of the present invention, the preferred methods and materials are described.

As used herein, the term "crystallizing agent" refers to a substance that, when present in a living cell or medium containing enzymes that catalyze dehydrogenating reactions, can form crystals. The crystallizing agents of the present invention are tetrazolium salts. Tetrazolium salts are compounds with tetrazole, tetrazolyl, or tetrazolo- structure. The tetrazolium salt is an organic salt in which the organic portion contains one or two tetrazole rings, generally with aryl (especially phenyl or substituted phenyl) or naphthyl, substituents at various positions, particularly the 1, 2, 3, and 5 positions. Tetrazolium, salts with two tetrazole rings are typically coupled so as to provide a diphenyl group or naphthyl group with the tetrazole rings in the two para positions. Exemplary tetrazolium compounds of this invention include the following: pABT (p-Anisyl Blue Tetrazolium Chloride); pApNBT, p-Anisyl-p-Nitro Blue Tetrazolium Chloride; BSPT, Thiazolyl blue (2-2'-Benzothiazolyl-5-styryl-3-(4'-phthalhydrazidyl) tetrazolium chloride); BT, Blue tetrazolium chloride; BTSPT, 2-(2'-Benzothiazolyl)-5-stylyl-3-(4'-phthalhydrazidyl)-tetrazolium chloride; CTC, (5-Cyano-2,3-ditolyl tetrazolium chloride); DMDPT, [3-(4,5-Dimethylthiazol-2-yl)-2,5-diphenyl]tetrazolium Bromide, 1-[4,5-dimethylthiazol-2-yl]-2,5-diphenyltetrazolium bromide; DSNBT, Distyryl nitroblue tetrazolium chloride; (1H)-tetrazole; IDNTT, Iodonitrotetrazolium chloride; INT, Iodo Nitro Tetrazolium Violet Chloride, p-iodo nitrotetrazolium violet (2-(4-iodopbenyl)-3-(4-nitrophenyl)-5-phenyltetrazolium chloride; INpT, 2-(p-iodophenyl)-p-nitrophenyl-5-phenyltetrazolium chloride; mNBT, m-Nitro Blue Tetrazolium Chloride; mNNT, m-Nitro Neotetrazolium Chloride; MNSTC, 2,2-bis(2-methoxyl-4-nitro-5-sulfophenyl)-2H-tetrazolium-5-carboxanilide; MTS: 3-(4,5-dimethylthiazol-2-yl)-5-(3-carboxymethoxyphenyl)-2-(4-sulfophenyl)-2H-tetrazolium salt; MTT, tetrazolium bromide, thiazolyl blue tetrazolium bromide, (3->4,5-dimethylthiazol-2-yl!-2,5-diphenyltetrazolium bromide); NBMT, Nitro Blue Monotetrazolium Chloride; NBT, p-Nitro Blue Tetrazolium Chloride, Nitro blue tetrazolium chloride (2,2'-di-nitrophenyl-5,5'-diphenyl-3,3'-(3,3'-dimethoxy-4,4'-diphenylene)ditetrazolium chloride); NT, Neotetrazolium chloride (2,2',5,5'-Tetraphenyl-3,3'(p-diphenylene)-ditetrazolium chloride; NTV, Nitrotetrazolium Violet; Thiazolyl blue; TB, tetrazolium blue chloride (3,3'->3,3'-dimethoxy(1,1'-biphenyl)-4,4'-diyl]-bis(2,5-diphenyl-2H-tetraz olium)dichloride); NBT, Nitroblue tetrazolium chloride; oTTR, o-Tolyl Tetrazolium Red; PCTMB, sodium 3'-[1-[(phenylamino)-carbonyl]-3,4-tetrazolium]-bis(4-methoxy-6-nitro)benzene-sulfonic acid hydrate; PNBT, p-Nitro Blue Tetrazolium Chloride; PTB, Piperonyl Tetrazolium Blue; pTTR, p-Tolyl Tetrazolium Red;TC-NBT, Thiocarbamyl nitro blue tetrazolium chloride (2,2'-di-p-nitrophenyl-5,5'-di-p-thiocarbamylphenyl-3,3'[3,3'-dimethoxy-4,4'-biphenylene]-ditetrazolium chloride; TNBT, Tetranitroblue tetrazolium chloride; TPTT, 1,3,5-triphenyltetrazolium; TR, TTC, TPT, Tetrazolium Red (2,3,5-triphenyltetrazolium chloride); TV, Tetrazolium violet, Violet Tetrazolium, 2,3,5-Triphenyl-2-H-tetrazolium chloride, 2,5-diphenyl-3-[.alpha.-naphthyl]-tetrazolium chloride, 2,5-diphenyl-3-[1-naphthyl]-2H-tetrazolium chloride; VTB, Veratryl tetrazolium blue; WST-1: 4-[3-(4-iodophenyl)-2-(4-nitrophenyl)-2H-5-tetrazolio]-1,3-benzene disulfonate; XTT, 2,2-bis(2-methoxyl-4-notro-5-sulfophenyl)-2H-tetrazolium-5-carboxanilide. Preferably, the crystallizing agent is selected from one or more member of the group consisting of tetrazolium violet, CTC, MTT, Tetrazolium Blue, HTT, tetrazolium red, TPF, ITT, INT, INPT, neotetrazolium chloride, NBT, TNBT, TC-NBT, TTD, MTS, WST-1, XTT, DDTT, and PCTT. Most preferably, the crystallizing agent is selected from one or more member of the group consisting of tetrazolium violet, CTC, MTT, Tetrazolium Blue, and tetrazolium red.

As used herein, the term "enzyme" refers to a substance that, when present in a living cell or medium containing their substrates, catalyzes dehydrogenating reactions. Enzymes can function in several ways: by dehydrogenating substances such as succinic acid or succinate (hereinafter referred to as "substrate") to provide cellular energy, or by catalytically converting salts such as tetrazolium salts to related formzan crystals. The enzymes that may catalyze the formation of crystals include, but are not limited to, oxidation reductases, DT-diaphorase, alcohol dehydrogenase, beta-hydroxysteroid dehydrogenase, inosine monophosphate dehydrogenase, glucose .alpha.-dehydrogenase, glucose-6-phosphate dehydrogenase, glyceraldehyde-3-phosphate dehydrogenase, glycerol-3 phosphate dehydrogenase (mGPDH), malate dehydrogenase, 3-.alpha.-hydroxysteroid dehydrogenase, lactate dehydrogenase, L-glutamate dehydrogenase, leucine dehydrogenase, aldehyde dehydrogenase, sarcosine dehydrogenase, amine dehydrogenase, succinate dehydrogenase, choline dehydrogenase, fructose dehydrogenase and sorbitol dehydrogenase. Preferably, such enzymes include inosine monophosphate dehydrogenase, telomerase, glucose-6-phosphate dehydrogenase, malate dehydrogenase, lactate dehydrogenase, and succinate dehydrogenase. Preferably, the enzyme is a dehydrogenase such as, but not limited to, SDH, inosine monophosphate dehydrogenase, glucose-6-phosphate dehydrogenase, malate dehydrogenase, and lactate dehydrogenase. More preferably, the enzyme is SDH, and lactate dehydrogenase.

As used herein, the term "organelle" refers to a subcellular unit or a subcellular structure defined by plasma membrane. The organelle in this invention includes, but (*is*) not limited to, nucleus (containing most of the cellular DNA and being the site of synthesis of cellular RNAs), nuclear matrix, nuclear lamina, core filaments, nuclear envelope (NEs), nuclear pore complexes (NPCs), and nuclear membranes. The organelle in this invention also includes mitochondrion, endoplasmic reticula (ER, a network of membranes in which glycoproteins and lipid are synthesized), Golgi complexes or Golgi apparatus (directing membrane constituents to appropriate places), lysosomes (degrading proteins, nucleic acids and lipids), and vacuoles (large fluid-filled organelles that store many nutrient and waste molecules, and also participate in the degradation of cellular proteins and other micromolecules). The organelle in this invention further includes chromosomes, nucleosome, chromatin, centromere, centrosome, centrioles, pericentriolar material (PCM), mitotic spindle, spindle pole bodies (SPBs), contractile rings, kinetochore, proteasomes, telomere, plasma membranes, endosomes, peroxisomes (metabolizing hydrogen peroxide), phagosomes, and ribosomes. Each type of organelle plays a unique role in the growth, metabolism, and metastasis of tumor cells, and each contains a collection of specific proteins or enzymes that catalyze the requisite chemical reactions. Some of this specificity resides in the organelle membranes, to which a number of the enzymes and other proteins are bound. In this invention, dehydrogenases, particularly succinate dehydrogenase (SDH), are found to be highly expressed in such organelles of a variety of cancer cells. Through the SDH-catalyzed dehydrogenating reaction, some chemicals such as tetrazolium salts can be reduced to the related formazan crystals. The crystallization of the organelles and/or cytoskeleton that are rich in SDH will have their functions and structures destroyed (frozen). The proteins or enzymes that located in the structures that are rich in SDH will be inactivated or frozen.

As used herein, the term "cytoskeleton" refers to a complex network of protein filaments traversing the cell cytoplasm. It is not simply a passive feature of the cell that provides structural integrity, it is a dynamic structure that is responsible for whole-cell movement, changes in cell shape, and contraction; it provides the machinery to move organelles from one place to another in the cytoplasm. Some cytoskeletal fibers may connect to organelles or provide tracks along which organelles and/or small molecules move. It is recently evidenced that the cytoskeleton is a master organizer of the cell's cytoplasm, furnishing binding sites for the specific localization of ribonucleic acids (RNAs) and proteins that were once thought to diffuse freely through the cytoplasm. Cytoskeleton is also responsible to the signal transduction. Activities of the cytoskeleton depend mainly on just three principal types of protein assemblies: actin filaments, microtubules, and intermediate filaments. Each type of filament or microtubule is formed from specific association of protein monomers. The dynamic aspects of the cytoskeletal structures arise from accessory proteins that control the length of the assemblies, their position within the cell, and the specific-binding sites along the filaments and microtubules for association with protein complexes, organelles, and the cell membrane. Thus, the present disclosure also includes the crystallization of these proteins, protein monomers, and protein filaments. Dynamically, crystallization of microtubules, centrosomes, and other cytoskeletons may interfere with the message (or signal) transmission or cell motility, and consequently make the cells lacking in mitosis motive force. The crystallization of nuclear envelopes may block the duplication of chromatins, and therefore obstruct the formation of daughter cells.

As used herein, the term "chemotherapeutic agent" refers to a chemical compound or composition capable of inducing cell death in a cell culture or a mammalian host tissue when properly administered to the host tissue. Chemotherapeutic agents that may be utilized in the present invention include any known chemotherapeutic agent or drug, or any combination of chemotherapeutic agents or drugs, which achieves its (*achieves a*) cytotoxic effect at least in part through interfering with cellular structure and/or metabolism. Suitable chemotherapeutic agent may be derived from any source, and include synthetic, semi-synthetic, naturally occurring, and recombinant compounds. Examples of conventional chemotherapeutic agents include, without limitation, platinum-compound such as cisplatin, carboplatin and their analogs and derivatives; alkylating agents such as chlorambucil, nitrogen mustards, nitromin, cyclophosphamide, 4-hydroperoxycyclophosphamide, 2-hexenopyranoside of aldophosphamide, melphalan, BCNU, antimetabolites such as 5-fluorouracil, folic acid, methotrexate (MTT, 6-mercaptopurine, aminopterin, 8-azaguanine, azathioprine, uracil, cytarabine, and azaserine; plant components such as cyclophosphamide, 4-hydroperoxycyclophosphamide, thiotepa, daunorubicin and neocarzinostain; antibiotics such as bleomycin, daunomycin, cyclomycin, actinomycin D, mitomycin C, carzinophylin, macrocinomycin, neothramycin, macromomycin, nogaromycin, cromomycin, 7-o-methylnogallol-4'-epiadriamycin,4-demethoxydaunorubicin, streptozotocin DON and mitozanthron; bis-chloroethylating agents, such as mafosfamide, nitrogen mustard, nornitrogen mustard, melphalan, chlorambucil; hormones such as estrogens; bioreductive agents such as mitomycin C and others such as mitoxantrone, procarbazine, adriblastin, epirubicin, prednimustine, ifosfamid. P-glycoprotein inhibitors such as thaliblastine and protein kinase inhibitors such as protein kinase C inhibitor (ilmofosine). Chemotherapeutic agents particularly refer to antimetabolites. These chemotherapeutic agents may be used either alone or in combination. Preferably, one antimetabolite and one alkylating agent are combined, and more preferably, cisplatin, chlorambucil, cyclophosphamide, bleomycin, or 5-fluorouracil which have different tumor killing mechanisms are combined. As new chemotherapeutic agents and drugs are identified and become available to the art, they may be directly applied to the practice of the present invention.

As used herein, the term "pharmaceutically acceptable" refers to a carrier medium, which does not interfere with the effectiveness of the biological activity of the active ingredient(s) and is not toxic to the host to which it is administered.

As used herein, the term "therapeutically effective amount" as applied to the crystallizing agent or chemotherapeutic agent refers to the amount of the component in the composition or administered to the host that results in an increase in the therapeutic index of the host. The "therapeutic index" can be defined for purposes herein in terms of efficacy (extent of tumor reduction). Suitable concentrations of the crystallizing agent can be determined by various methods, including generating an empirical dose-response curve, predicting potency and efficacy using QSAR methods or molecular modeling, and other methods used in the pharmaceutical sciences. Similarly, suitable concentrations of the chemotherapeutic agent can be determined using conventional methods.

As used herein, the term "anticancer therapy" refers to an anticancer treatment or combination of treatments that depends, at least in part, on interfering cellular structures and/or metabolism. Anticancer therapies that may be utilized in the present invention include any known anticancer methods that achieve their anticancer effects at least in part through interfering cellular structures and/or metabolism. Suitable anticancer therapies of the present invention include, without limitation, radiation therapy, immunotherapy, chemotherapy, hormone therapy, hyperthermia therapy, photodynamic therapy, electrotherapy, gene therapy, antisense therapy, hyperbaric oxygen treatment, ischemia/reperfusion therapy, and the like. As new anticancer therapies are identified and become available to the art, they may be directly applied to the practice of the present invention.

As used herein, the term "effective dose" or "therapeutically effective dose" as applied to the anticancer therapy refers to the amount or unit dose of the anticancer therapy that results in an increase in the therapeutic index of the host. Suitable doses of the anticancer therapy can be determined using well-known methods, a variety of which are known and readily available in the pharmaceutical sciences.

As used herein, "conjointly" and "concurrently" mean at a time and in an amount such that the desired sensitizing effect of the crystallizing agent is in existence or comes into existence when the primary treatment effect of the anticancer therapy is taking place.

As used herein, the term "cancer or tumor" refers to any neoplastic disorder, including carcinomas, sarcomas and carcino-sarcomas. Specific types of cancers include, without limitation, glioma, gliosarcoma, anaplastic astrocytoma, medulloblastoma, lung cancer, small cell lung carcinoma, cervical carcinoma, colon cancer, rectal cancer, chordoma, throat cancer, Kaposi's sarcoma, lymphangiosarcoma, lymphangioendotheliosarcoma, colorectal cancer, endometrium cancer, ovarian cancer, breast cancer, pancreatic cancer, prostate cancer, renal cell carcinoma, hepatic carcinoma, bile duct carcinoma, choriocarcinoma, seminoma, testicular tumor, Wilms' tumor, Ewing's tumor, bladder carcinoma, angiosarcoma, endotheliosarcoma, adenocarcinoma, sweat gland carcinoma, sebaceous gland sarcoma, papillary sarcoma, papillary adenosarcoma, cystadenosarcoma, bronchogenic carcinoma, medullar carcinoma, mastocytoma, mesotheliorma, synovioma, melanoma, leiomyosarcoma, rhabdomyosarcoma, neuroblastoma, retinoblastoma, oligodentroglioma, acoustic neuroma, hemangiablastoma, meningioma, pinealoma, ependymoma, craniopharyngioma, epithelial carcinoma, embryonic carcinoma, squamous cell carcinoma, base cell carcinoma, fibrosarcoma, myxoma, myxosarcoma, liposarcoma, chondrosarcoma, osteogenic sarcoma, leukemia, lymphoma, and the metastatic lesions secondary to these primary tumors. In general, any neoplastic lesion, including granuloma, may be treated according the present invention. Therefore, the "cancer" in this invention also includes the cancer supporting components such as tumor angiogenesis, and tumor endothelial cells.

A basis of the present invention is the unexpected finding that tetrazolium salts exhibit potent anticancer activity both in vitro and in vivo. While the mechanism of action is not fully understood, and not wishing to be bound by any particular theory, it is believed that these agents exert their anticancer effect through formation of visible crystals on the key cellular structures including organelles and/or cytoskeleton. The formation of crystals can reduce the ability of tumor cell to provide energy (mitochondrial crystallization for example) and cease cell division of the targeted cells (crystallization of centrosomes, microtubules, and nuclear envelope, for example) as well. The crystallization of centrosome, the said cell brain in the present disclosure, will deactivate many key enzymes located on it.

The key decision determining whether or not a cell will divide is the decision to enter the S phase. Once a cell has become committed to enter S phase, it enters S phase several hours later and progresses through the remainder of the cell cycle until it completes mitosis. Cell replication is primarily controlled by regulating the time of two critical events in the cell cycle: the initiation of nuclear DNA, synthesis and the initiation of mitosis. Therefore, the real "coordinator", if any, as described in the previous section of this disclosure, should be the organelles that send signals to trigger the initiation of S phase and/or mitosis.

Based on this consideration, the inventor investigated the mechanisms through which the S phase is initiated. Unexpectedly, the whole centrosomes of the cells of varying cancerous origins treated with tetrazolium violet were specifically crystallized. DNA analysis demonstrated that most of the treated cells were blocked irreversibly in G₁ phase in a dosage dependent manner. The ease of DNA replication can be interpreted in two ways. The first is that DNA damage induces checkpoints that prevent entry into the S phase and M phase. The second, and most appealing, possibility is that the frozen centrosomes become unable to send, activate or concentrate some essential factor(s) required to induce DNA replication. The first possibility may be ignored, for no apparent DNA damage and no obviously altered M phase were observed. Unexpectedly, the cells with the frozen centrosomes demonstrated a new kind of death differing from either necrosis or apoptosis (programmed cell death). For the purpose of clarity, the cellular-organelle-crystallization-induced-death is referred to as "COCID".

In the further experiments, adriamycin that was previous reported to induce cell cycle arrest before S phase was exposed to cells in comparison with COCID inducing agents (e.g. tetrazolium violet). DNA damage and apoptosis with moderate S phase arrest were observed in the cells treated with adriamycin, as reported by other. The cells treated with tetrazolium violet were mostly arrested before S phase but no apoptosis was observed. Consistently, irradiation that was reported to induce S phase arrest also induced apoptosis. More surprisingly, preincubation of cells with tetrazolium violet significantly reduced the apoptosis induced by either adriamycin or irradiation in a dosage dependent manner. Therefore, the inventor of this invention believe that the centrosome is required not only for cell cycle progress through G₁ into S phase but also for apoptosis regulation. The implication of the centrosomes in S phase and in apoptosis observed by the inventor of this invention, which has not been reported by others, experimentally confirmed the cell brain theory postulated in this disclosure. Most surprisingly, the crystallization and growth inhibition induced by crystallizing agents (such as tetrazolium violet) were not observed in normal cells (CHO cell line), and were significantly inhibited by dehydrogenase inhibitors in the tested cancer cells.

To investigate the effect of the centrosome on M phase regulation, the microtubules of varying cancerous origins were frozen with MTT with the cellular DNA analyzed with flow-cytometry. Surprisingly, the cells were irreversibly blocked in G₂M phase in a dose dependent manner. Considering the microtubule nucleating ability of the centrosome in mitosis, the inventor believes that the centrosome is also responsible for mitosis regulation through some unknown mechanisms.

In summary, the findings that cells were irreversibly blocked in S phase by centrosome crystallizing agent (e.g.tetrazolium violet), and that the cells were irreversibly blocked in G₂M phase by microtubule crystallizing agent (e.g. MTT) both in a dose dependent manner, together with the regulating effect of the centrosome in apoptosis provide strong evidence supporting the cell brain theory of this disclosure. The crystallization of the centrosome and/or microtubules not only ceases DNA replication and mitosis but also causes mechanical injury of the targeted cells. The crystallization of centrosomes, microtubules and nuclear envelopes further interfere with the mobility of the cell and the transmission of cellular messages. Due to the decrease of the intracellular concentration of endogenous enzymes such as succinate dehydrogenases in the treated tumor and the freezing of the cellular cytoskeletons, cancer cells become highly susceptible to the damage induced by other treatments, particularly agents that interfere with cellular structures, cell cycles and metabolism.

In one aspect of the invention, a tetrazolium salt as a crystallizing agent is used in a therapeutic composition to treat tumor cells. While it is possible to administer the crystallizing agent(s) alone, it is believed preferable to present it as part of a pharmaceutical formulation. Such pharmaceutical formulations are preferably for local use, and most preferably for intralesional administration. In accordance with this aspect of the invention, the pharmaceutical compositions comprise at least one tetrazolium salt as a crystallizing agent in a therapeutically effective dose together with one or more pharmaceutically acceptable carriers and optionally other therapeutic ingredients. Preferred carriers include inert, non-toxic solids (e.g., dextrose, cellulose, starch), semi-solids (e.g., glycerol sterate, polyethylene glycol, stearic acid, and propylene glycol) and liquids (e.g., buffered saline, water, ethanol). The preferred form depends on the intended mode of administration. For example, injectables can be prepared as liquid solutions or suspensions, solid forms suitable for solution or suspension in liquid prior to injection, or as emulsions. A wide variety of pharmaceutically acceptable carriers are known to those of skill in the art. If desired, the pharmaceutical composition may also contain minor amounts of nontoxic auxiliary substances such as wetting or emulsifying agents, pH buffering agents, and the like, Compositions comprising such carriers and adjuvants may be formulated, and the resulting formulations may be sterilized, using well-known conventional methods and techniques. Methods for administration are also well known in the art.

One or more crystallizing agents may be loaded in a biocompatible polymer matrix body such that a slow or sustained, preferably constant, release of the crystallizing agent(s) is achieved. The polymer matrix substrate may be formed of any of a number of biostable or biodegradable polymers that act as the carrier matrix for the crystallizing agent(s). Preferably, the biocompatible polymer matrix body is comprised of a biodegradable, resorbable polymeric material. A wide variety of suitable biodegradable polymers are known and readily available to the art including, for example, polylactic acid, poly (lactideglycolide) copolymers, polyanhydrides, cyclodextrans, polyorthoesters, n-vinyl alcohol, and other biosorbable polymers. In the examples herein, the biodegradable polymer is a polylactic acid (PLA) polymer having a molecular weight of 20,000 or 100,000. Methods of preparing impregnated biocompatible polymers are known in the art. The impregnated biocompatible polymer can be surgically implanted into the tumor (i.e., intralesional administration) using well-known techniques. The intralesional administration of chemotherapeutic drugs using impregnated biodegradable polymers is described in (Kong, Q., et al., (1997) J Surg Oncol 64:268-273; Kong, Q., et al., (1998) J Surg Oncol 69:76-82).

A therapeutically effective amount of a tetrazolium salt as a crystallizing agent may be provided for administration to a mammalian host, preferably a human host, to treat cancer. The required dosage will depend upon the nature of the cancer, the severity and course of the cancer, previous therapy, the patient's health status, the particular crystallizing agent or combination of agents, and the judgment of the treating physician. In general, a suitable effective dose of the crystallizing agent will be in the range between about 0.001 to about 1,000 milligram (mg) per kilogram (kg) of body weight of recipient per treatment, preferably in the range between about 1.0 to about 100 mg per kg of body weight per treatment. The dose may be administered through any available approaches, which include but are not limited to intravenous, intra-artery, intra-cavity, intraperitoneal, and subcutaneous ways. The dose is preferably administered intralesionally using a pharmaceutically acceptable carrier appropriate for the mode of administration, such as by implanting an impregnated polymeric matrix or by administering the dose in a buffered saline solution through a catheter continuously or at appropriate intervals throughout the day. When the tumor has been eradicated or reduced to an acceptable size, a maintenance dose can be administered, if necessary. Subsequently, the dosage and/or the frequency of administration can be reduced, or treatment can cease. Patients can, however, require intermittent treatment on a long-term basis upon any recurrence of the cancer.

A further basis of the present disclosure is the discovery that the therapeutic index of an anticancer therapy can be enhanced in in vitro and in vivo systems by concomitantly or separately treating the host with a crystallizing agent. Specifically, the present inventors have determined that crystallizing agents increase the sensitivity of tumor cells to a wide variety of anticancer therapies, namely those that depend, at least in part, on interfering with cellular structures and/or metabolism for their anticancer activity. Unexpectedly, crystallizing agents were found to potentiate the damage the tumors suffered when exposed to non-surgical anticancer treatments. Non-surgical anticancer procedures include radiation therapy, hyperthermia therapy, photodynamic therapy, electrotherapy, gene therapy, antisense therapy, and ischemia/reperfusion therapy. The present inventor's findings also suggest that crystallizing agents may be useful for enhancing or potentiating tumor damage caused by chemotherapeutic agents and drugs, immunotherapy, gene therapy, and hormone therapy. While the mechanism is not fully understood, and not wishing to be bound by any particular theory, it is believed that the crystallizing agents may exert anticancer effects by reducing the ability of the tumor cells to provide energy, by damaging cellular structures and/or interfering with signal transduction and other cellular procedures including but not limited to cell cycle regulation, DNA synthesis, DNA replication, transcription regulation, and mitosis. As far as the agents that crystallizes the centrosome and/or centrioles crystallization are concerned, the said cell brain as a whole in this disclosure might be targeted Because the intracellular and/or interstitial concentrations of endogenous enzymes such as succinate dehydrogenase in the treated tumor are effectively reduced or exhausted, the tumor cells become highly susceptible to the damages induced by the primary treatments. Moreover, it is believed that the use of tetrazolium salts as crystallizing agents in accordance with the invention will also minimize or eliminate the chemotherapy-induced tumor resistance (commonly referred to as "multidrug resistance") which are typically associated with conventional anticancer therapies. Specifically, the crystallization of the cell brain of this disclosure, all cellular activities will be shut down irreversibly.

Accordingly, the present disclosure provides a method for treating tumor cells comprising administering a crystallizing agent in an amount effective for sensitizing the cells to other anticancer therapy. The administration of a crystallizing agent increases the sensitivity of tumor cells to a wide variety of non-surgical cancer therapies, such as those listed above. Crystallizing agents may be used for the treatment of various forms of malignant diseases, particularly melanomas, adenomas, sarcomas, leukemia, lymphomas, hypoxic tumors, carcinomas of solid tissues, and osteogenic sarcomas. Crystallizing agents may also be used for the treatment of other neoplastic growth such as angiomas and granulomas. Treatment with crystallizing agents may also allow for more effective anticancer therapies of tumors that currently respond poorly to conventional treatments, including tumors, which have developed a resistance to such therapies. When the crystallizing agent is used to potentiate or enhance the damage to tumor cells by conventional anticancer therapies, the crystallizing agent or the combination of crystallizing agents is generally administered prior to or simultaneously with administration of the anticancer therapy, i.e., "conjointly" with the primary therapy. Specifically, when used with gene therapy, the enzymes such as SDH are preferably up regulated prior to the administration of the said crystallizing agents. The methods for up regulation of the enzymes are known in the art to the skilled person.

As will be appreciated by those skilled in this art, the required dose or "effective dose" of the anticancer therapy to be utilized in treating tumors will depend on a variety of factors. Such factors include the nature of the anticancer therapy, the nature of the cancer, the severity and course of the cancer, previous therapy, the patient's health status, the particular crystallizing agents or combination of agents, and the judgment of the treating physician. In general, the dosages of the particular therapy to be used in the present invention are fixed by the particular requirements of each situation. However, the presence of the crystallizing agent increases the number of tumor cells that are killed by the anticancer therapy. Thus, the standard, pre-set dosage of the anticancer therapy is expected to become more effective in the presence of the crystallizing agents than in their absence.

According to the present disclosure, a therapeutically effective amount of a crystallizing agent may be administered to a mammalian host, preferably a human host, to selectively sensitize the tumor cells during cancer therapy. The method comprises administering to a host having cancer a crystallizing agent in an amount effective for selectively sensitizing the cancer cells to the injury, The present disclosure also provides a method for selectively killing cancer cells in a mammalian host, preferably a human host. The method for selectively killing cancer cells comprises conjointly administering to the host a crystallizing agent and a dose of an anticancer therapy sufficient to selectively kill the cancer cells without killing substantial numbers of normal cells. Preferably, the crystallizing agent is a substrate of the enzymes. In a particularly preferred embodiment, the substrate of the enzymes is the substrate of succinate dehydrogenase. Examples of a substrate of succinate dehydrogenase include tetrazolium salts. Tetrazolium salts are compounds with tetrazole, tetrazolyl, or tetrazolo- structure. Examples of tetrazolium salts include, but are not limited to, MTT, CTC, TB, Tetrazolium Blue, HTT, tetrazolium red, TPF, ITT, INT, tetrazolium violet, INPT, neotetrazolium chloride, NBT, diformazan of NBT, TNBT, TC-NBT, TTD, MTS, WST-1, XTT, DDTT, and PCTT. The required dosage of the crystallizing agent will depend upon the nature of the anticancer therapy, the nature of the cancer, the severity and course of the cancer, previous therapy, the patient's health status, the particular crystallizing agent or combination of agents, and the judgment of the treating physician. In general, a suitable effective dose of the crystallizing agent will be in the range between about 0.001 to about 1,000 milligram (mg) per kilogram (kg) of body weight of recipient per treatment, preferably in the range between about 1.0 to about 100 mg per kg of body weight per treatment. When the tumor has been eradicated or reduced to an acceptable size, a maintenance dose of the crystallizing agent and/or cancer therapy can be administered, if necessary. Subsequently, the dosage and/or the frequency of administration can be reduced, or treatment can cease.

A tetrazolium salt as a crystallizing agent may be combined with at least one chemotherapeutic agent in a therapeutic composition to treat tumor cells. Pharmaceutical compositions may comprise one or more tetrazolium salts as crystallizing agents and one or more chemotherapeutic agents, each of the components being present in a therapeutically effective amount, together with one or more pharmaceutically acceptable carriers and optionally other therapeutic ingredients. Preferred carriers include inert, non-toxic solids (e.g., dextrose, cellulose, starch), semi-solids (e.g., glycerol sterate, polyethylene glycol, stearic acid, and propylene glycol) and liquids (e.g., buffered saline, water, ethanol). Compositions comprising suitable carriers and adjuvants can be formulated, prepared, and administered using conventional, well-known methods. Preferably, the crystallizing agent(s) and chemotherapeutic agent(s) are loaded in a biocompatible polymer matrix body such that a slow or sustained, preferably constant, release of the crystallizing agent(s) is achieved. The polymer matrix body may be formed of any of a number of biostable or biodegradable polymers that act as the carrier matrix for the crystallizing agent(s), also as discussed above. Preferably, the biocompatible polymer matrix body is comprised of a biodegradable, resorbable polymeric material and a wide variety of which are known and readily available to the art. The impregnated biocompatible polymer can be surgically implanted into a tumor using well-known techniques.

Chemotherapeutic agents suitable for use in the compositions of the present invention may be any known pharmaceutically acceptable agent that depends, at least in part, on interfering with cellular structure and/or metabolism for its anticancer activity. Preferably, one anti-metabolite and one antimicrotubule agent are combined, and more preferably, taxol, cisplatin, chlorambucil, cyclophosphamide, bleomycin, or 5-fluorouracil which have different tumor killing mechanisms are combined. The combination containing arsenic compounds, colchicin, colchicine, colchiceine, colchisal, colchium salts, vinblastine, paclitaxel and related compounds that interfere with the cytoskeletons are most preferred. As new chemotherapeutic agents and drugs are identified and become available to the art, they may be directly applied to the practice of the present invention.

In addition, crystallizing agent(s) with or without chemotherapeutic agents can be combined with the known non-surgical anticancer techniques such as radiation, photodynamic therapy, gene therapy, antisense therapy, immunotherapy, hyperthermia, and electric therapy.

It was also found that crystallizing agent(s) and/or chemotherapeutic agents might stimulate tumor growth at low dosages. In addition to stimulating tumor growth, low dose therapies also tend to induce resistance to subsequent treatments (Kong, Q., et al., (2000) *supra*). Thus, a significant advantage of the present invention is the ability to effectively deliver high dosages of a tetrazolium salt as a crystallizing agent(s) with or without chemotherapeutic agents through the local, preferably intralesional, administration of crystallizing agent(s) with or without chemotherapeutic agents. In one arrangement, a biocompatible polymeric matrix device is used to provide a slow, sustained release of the active ingredient(s). Such devices not only deliver a high drug concentration to the tumor for an extended period of time (thereby enhancing the cytotoxic effect), but also minimize crystallizing agent(s)-related side effects (i.e., systemic toxicity).

In addition to the treatment of cancer, said crystallizing agents may be used for the treatment of patients with other infections such as, including, but not limited to, viral, bacterial, fungal, and parasitic infections.

According to the disclosure, one or more crystallizing agents are used to visualize cellular organelles or cytoskeletons in a tissue or cells. While it is possible to use one crystallizing agent, it is believed preferable to use more crystallizing agents. The combination of more than one agents is preferable for visualizing cells, and most preferable for differentiating cancer cells from the normal ones. Preferably, the crystallizing agent is a substrate of the enzymes. In a particularly preferred embodiment, the substrates of the enzymes are the substrates of succinate dehydrogenase. Examples of the substrates of succinate dehydrogenase are tetrazolium salts and their analogues or derivatives. Tetrazolium salts are compounds with tetrazole, tetrazolyl, or tetrazolo-structure. Examples of tetrazolium salts include, but are not limited to, MTT, TB, Tetrazolium Blue, HTT, tetrazolium red, TPF, ITT, INT, tetrazolium violet (TV), INPT, neotetrazolium chloride, NBT, diformazan of NBT, TNBT, TC-NBT, TTD, MTS, WST-1, XTT, DDTT, and PCTT. The combination of the crystallizing agents is also determined by the targets that are to be detected. For example, tetrazolium violet (TV) forms crystals mainly in centrosome, therefore it can be used to specifically visualize centrosomes, while MTT forms crystals in microtubules, mitochondrion, and nuclear envelopes; and TR forms crystals in envelopes and/or other structures close to envelopes such as endoplasmic reticula. Therefore different tetrazolium salts or analogues may specifically crystallize certain cellular organelles or sub-organelles.

An effective amount of a crystallizing agent may be administed to a cell sample or tissue sample, preferably a cell sample, to visualize cellular organelles and/or cytoskeletons of cells. The required dosage will depend on the nature of the cell, contacting period of time, cell number, thickness of tissue section, crystallizing agents that will be used, and the visualizing targets. In general, a suitable effective dose of the crystallizing agent will be in the range of about 0.1 to about 1000 microgram (ug) per milliliter (ml), preferably in the range of about 1.0 to about 100 ug per milliliter. The dose is preferably administered directly into the medium containing cells or tissue sample.

One or more crystallizing agents may be used in a kit to visualize cellular organelles and cytoskeletons. While it is possible to use the crystallizing agent(s) alone, it is believed preferable to present it as part of a formulation. Such formulations are preferably for visualizing cells, and most preferably for differentiating cancer cells from the normal ones. In accordance with this aspect, the kits comprise at least one crystallizing agent in an effective dose together with one or more carriers and optionally other ingredients. Preferred carriers include inert, non-toxic liquids (e.g., culture media, buffered saline, water, culturing medium). If desired, the kit may also contain minor amounts of nontoxic auxiliary substances such as wetting or emulsifying agents, pH buffering agents, and the like. Compositions comprising such carriers and adjuvants may be formulated, and the resulting formulations may be sterilized, using well-known conventional methods and techniques. Methods for administration are also well known in the art. In addition to the visualization of mammalian cells, said crystallizing agents may be used for the visualization of other cells such as, but not limited to, a variety of virus, bacteria, fungus, and parasite cells under proper conditions. In addition, the formation of visible crystals alone or in combination with the functional and/or structural changes induced by the crystallizing agents, the crystallizing agents may be used to study other cellular activities such as cell cycle, signal transduction, aging, carcinogenesis, apoptosis, antigen editing, receptor regulation, memory, motility, biological clock, checkpoint, molecule transportation, intercellular reaction, gene transcription, intracellular action, developmental biology, cancer biology, degenerative disease, and autoimmune disease. The said cell brain, mainly consisting of the centrosome, centrioles and the connecting filaments, is expected to play an important role in regulating a cell's self defense and other cellular procedures.

One or more crystallizing agents may be used for isolating cellular organelles through the crystallization of the interesting organelles. Crystallization will effectively conserve the native 3-D structure, and therefore avoid the possibilities of artificially changing the structures of the isolated organelles by traditional techniques (Paintrand, M. et al., *supra*). The isolated organelles are used for structural studies of the organelles or to provide the organelle-associated proteins for the establishment of a protein bank, preferably a protein bank for the cell brain. The method for isolating cellular organelles and/or cytoskeletons, in tissue or in cell suspension comprising obtaining a tissue sample or cell sample that contains a plurality of cells; treating said tissue sample or cell sample with one or more crystallizing agents under conditions that permit formation of visible crystals through the reduction of said crystallizing agent by the enzymes in said cellular organelles and/or cytoskeletons of said cells or tissues; detecting the number and shapes of said cellular organelles and/or cytoskeletons, and isolating said cellular organelles and/or cytoskeletons through ingredient centrifugation. The isolated organelles are further used for studying the studying the structures of said organelles under proper conditions such as electronic microscope or for isolating and analyzing the proteins on them using known molecular or biochemical techniques. The tissue or cell is selected from the group consisting of normal mammalian tissue or cell, pathologic mammalian tissue or cell, and neoplastic tissue or cell. The tissue or cell can be from organisms such as but not limited to bacteria, parasite, fungus, yeast and plant. The cells are preferably genetically engineered cells. The crystallizing agent used in the isolation of cellular organelles may be any tetrazolium salts. Examples of said tetrazolium salts is selected from the group consisting of tetrazolium salts, 1H-tetrazolium, 2H-tetrazolium, tetrazolium violet, MTT. The procedures for isolation of cellular organelles, isolation of organelle proteins and the application of the proteins are known to the arts.

Except for isolating cellular organelles, crystallizing agents may be used for screening and detecting dehydrogenase inhibitors. Once the candidate dehydrogenase inhibitors are used, the crystallizing agents will not form visible crystals. The crystallizing agent used for this purpose may be any tetrazolium salt. It is preferably selected from the group consisting of tetrazolium salts, 1H-tetrazolium, 2H-tetrazolium, tetrazolium violet, MTT. The procedures for detecting or screening dehydrogenase inhibitors are known to biologists in the art.

The invention is further described by the following examples, which are illustrative of specific modes of practicing the invention and are not intended as limiting the scope of the invention as defined by the appended claims.

In the Examples, which follow, the biodegradable polymer was prepared in the following manner. Pre-weighed active agents and lactic acid were dissolved in dichloromethane. The resulting solution was then degassed using a vacuum pump for 5 to 24 hours. The nearly dry polymer was harvested for shaping, then further dried in an incubator at 10 to 37°C for approximately 20 to 40 hours. The dried polymer was sterilized using gamma-irradiation and stored in a dry, cool location until use.

The terms and abbreviations used in the instant examples have their normal meanings unless otherwise designated. For example "degree C" refers to degrees Celsius; "µl" refers to microliter or microliters; "µg" refers to microgram or micrograms; "mg" refers to milligram or milligrams; "ml" means milliliter or millliters. Unless specified otherwise, commercially available chemicals were used without purification. All scientific and technical terms have the meanings as understood by one with ordinary skill in the art. The specific examples which follow illustrate the in vivo and in vitro efficacy of certain representative compositions and are not to be construed as limiting the invention in sphere or scope.

The present invention is more specifically illustrated by the following Examples.

### EXAMPLES

### EXAMPLE 1: PREPARATION OF CELLS FROM FRESH TISSUES FOR CELLULARORGANELLE VISUALIZATION

Cell suspensions were removed from surgical resection specimens of intracranially or subcutaneously established gliosarcomas in rats by removing samples (0.5 - 1.0 cm square) of tissue and then mincing the samples with a razor blade in PBS at room temperature. Minced tissue was then washed in PBS and resuspended in 2 ml aliquot of PBS containing 2.0 U/ml of collagenase and 0.2 U/ml DNase , and then incubated for 2 hours at room temperature. During the incubation, the samples were rotated end over end. Following collagenase and DNase digestion, the samples were strained on a 100 micrometer nylon filter (Nytex, Small Parts, Inc., Miami Lakes, Fla.), pelleted, washed in PBS, by sequential centrifugation at 325 G. Then they were transferred into a flask for culture as described in Examples 2-7 (below). Cells can also be obtained from fresh tissue, peripheral blood, bone marrow, and lymph nodes and treated in a similar manner.

### EXAMPLE 2: EFFECT OF CRYSTALLIZING AGENT ON 9L GLIOSARCOMA TUMOR CELLS

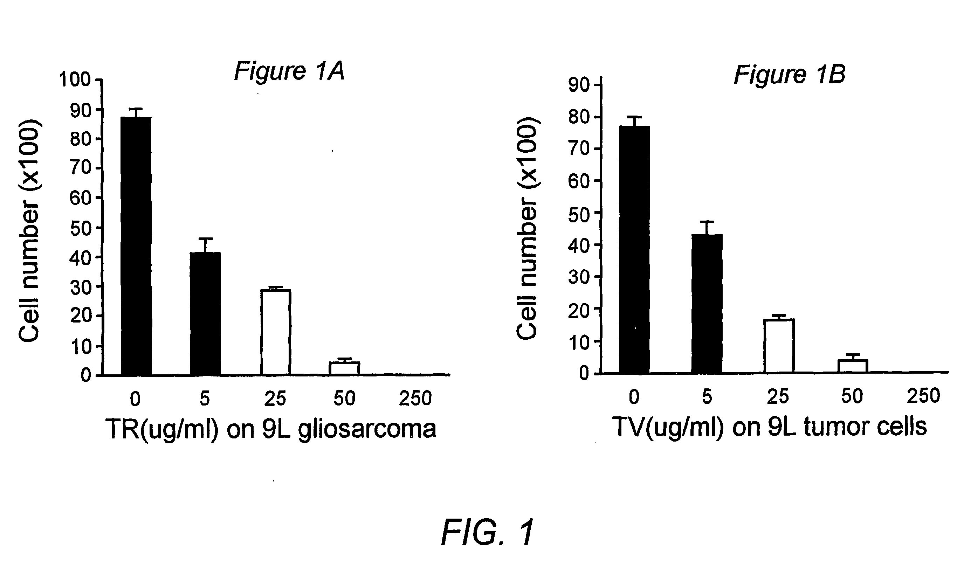

Tumor cells were exposed to escalating concentrations of SDH substrates, tetrazolium red (TR) and tetrazolium violet (TV), respectively. The tumor cells (4000) were seeded in a 48 well plate one day before treatment was administered. The concentrations of TR and TV were same, as indicated in Figures 1A and 1B, respectively. The concentration was 250, 50, 25, 5, and 0 µg/ml. Tumor cell number was calculated one day post treatment. Surviving tumor cell numbers are shown in Figures 1A (treated with TR) and 1B (treated with TV), respectively. These data demonstrate that tetrazolium salts, the substrates of SDH are effective, in a dose dependent manner, in killing tumor cells.

### EXAMPLE 3: SYNERGISTIC EFFECT OF CRYSTALLIZING AGENT AND CHEMOTHERAPEUTIC AGENTS ON 9L GLIOSARCOMA

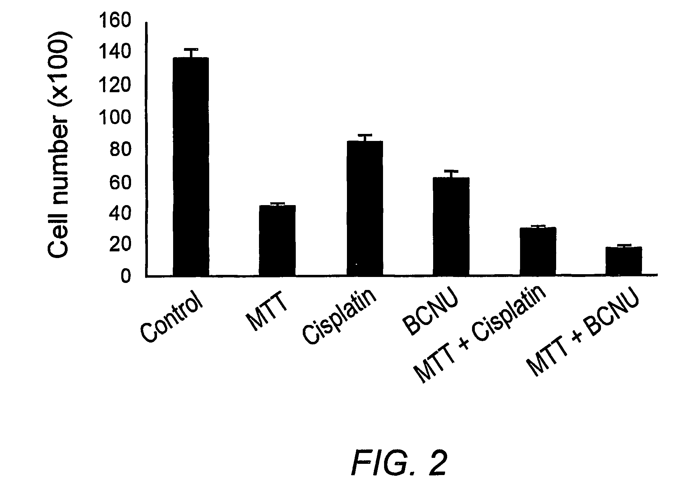

Crystallizing agents and various chemotherapeutic agents were tested, individually and in combination, on 9L gliosarcoma tumor cells. Treatment was administered one day after the cells (4000) were seeded in a 48 well plate, with the number of cells counted in 6 hours. MTT at 10 ug/ml was tested with cisplatin and BCNU. MTT at this dose significantly (p <0.005) enhanced the cytotoxic effects of cisplatin (10 µg/ml), and BCNU (25 µg/ml) (Figure 2). All data represent the mean of 3 to 4 repetitions.

As is evident from Figure 2, the crystallizing agent and chemotherapeutic agents are effective in inhibiting tumor cell growth when applied individually. However, the combination of crystallizing agent and chemotherapeutic agents is demonstrated to be synergistic.

### EXAMPLE 4: IN VIVO ANTITUMOR ACTIVITY OF CRYSTALLIZING AGENT

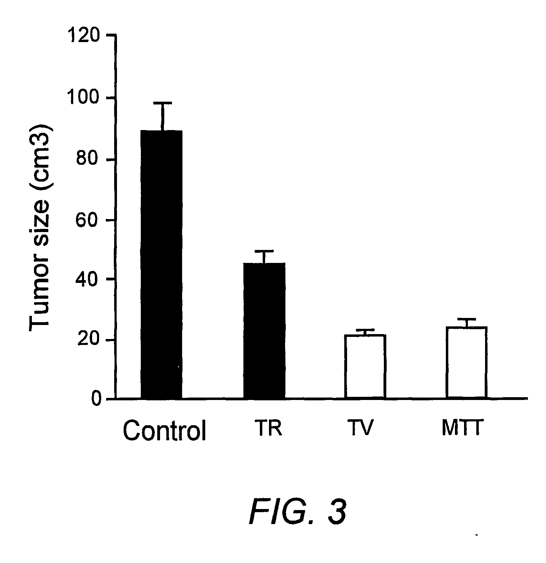

Small cell lung cancer (SCLC) cells (2x10⁵) were injected subcutaneously in rats to test the in vivo cytotoxicity and efficacy of tetrazolium salts. Tetrazolium salts in polylactic acid polymer were implanted in rats with 14 day established 9L gliosarcoma in the right flank area. Treatment was initiated as follows: G-1, tumor control; G-2, TR in polymer at 2.5 mg/kg; G-3, TV in polymer at 2.5 mg/kg; and G-4, MTT 2.5 mg/kg. The tumor volume was measured on day 60. The tumor size was significantly smaller (p < 0.05) in the treated groups, as compared to tumor control (88.5 ± 7.8 cm³). See Table 1 and Figure 3.

**Table 1**

| Group (n) | Treatment | Tumor size (cm³) | P values |
|---|---|---|---|
| 1 (6) | --- | 88.5±23 cm³ | |
| 2 (6) | TR, 2.5 mg/kg | 44±5.3 cm³ | <0.05 |
| 3 (6) | TV, 2.5 mg/kg | 21±2.3 cm³ | <0.01 |
| 4 (6) | MTT, 2.5 mg/kg | 24±3.6 cm³ | <0.01 |

### EXAMPLE 5: VISUALIZATION OF CENTROSOMES OF SHP77 (SCLC) CELLS FOLLOWING TREATMENT WITH TV.

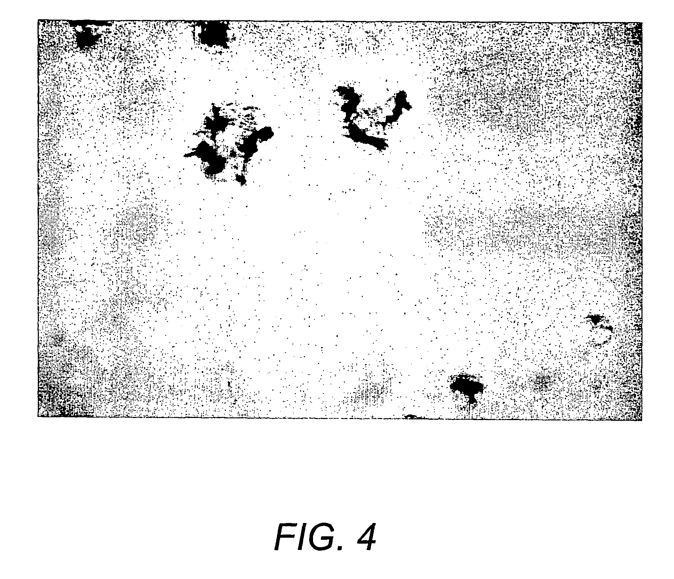

SHP77, small cell lung cancer cells (SCLC), were exposed to tetrazolium violet (TV, a substrate of SDH). The tumor cells (4000) were seeded in a 48 well plate one day before being treated. The concentration of TV was 50 µg/ml. As shown in Figure 4, centrosomes, usually one in each cell, were crystallized at the poles, with the cell viability being significantly reduced (see data in example 1). After being treated under the same condition, the centrosome of 9L gliosarcoma tumor cells were all crystallized (figures not shown).

### EXAMPLE 6: VISUALIZATION OF MICROTUBULES OF 9L GLIOSARCOMA CANCER CELLS FOLLOWING TREATMENT WITH MTT

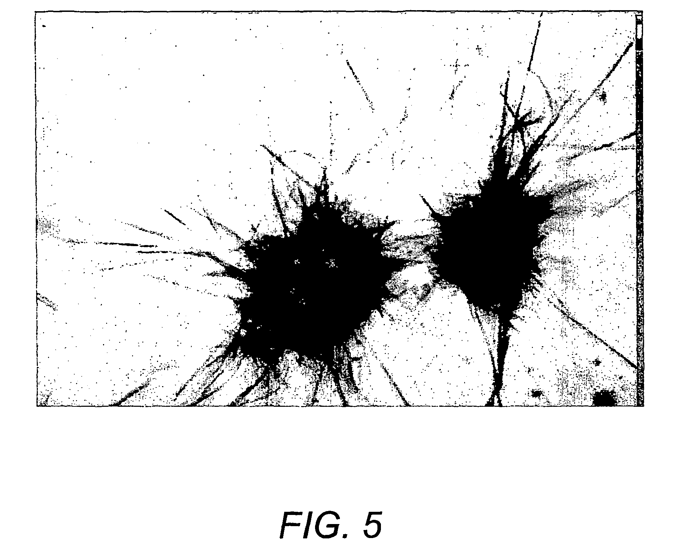

9L cancer cells were exposed to MTT (50ug/ml). The tumor cells (4000) were seeded one day before being treated with MTT. As can be seen Figure 5, the microtubules and nuclear envelopes were frozen by the crystals formed on them.

### EXAMPLE 7: CRYSTALLIZATION OF MITOCHONDRION OR LYSOSOMES OF 9L GLIOSARCOMA CELLS FOLLOWING TREATMENT WITH MTT

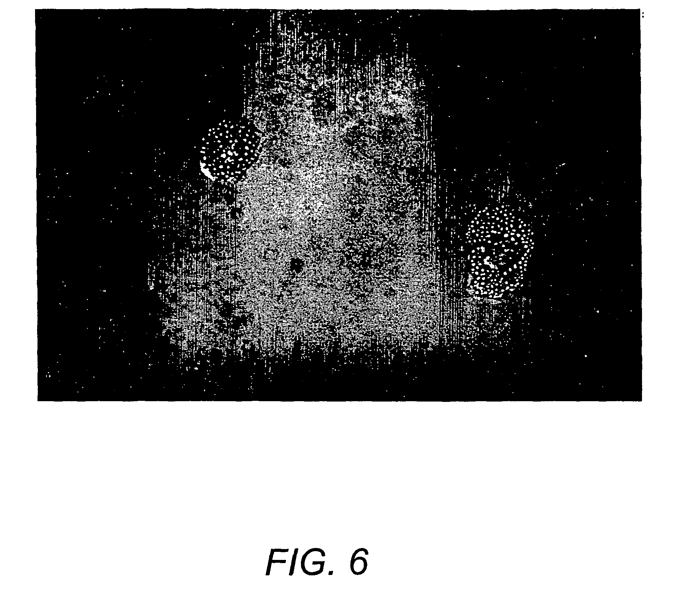

9L gliosarcoma cancer cells were exposed to MTT (50ug/ml)- a substrate of SDH. The tumor cells (4000) were seeded one day before being treated with MTT. As can be seen in Figure 6, the mitochondrion or lysosomes were crystallized by the crystals formed on them.

### EXAMPLE 8: CRYSTALLIZATION OF NUCLEAR ENVELOPES OR ENDOPLASMIC RETICULUM OF 9L GLIOSARCOMA CELLS FOLLOWING TREATMENT WITH TR

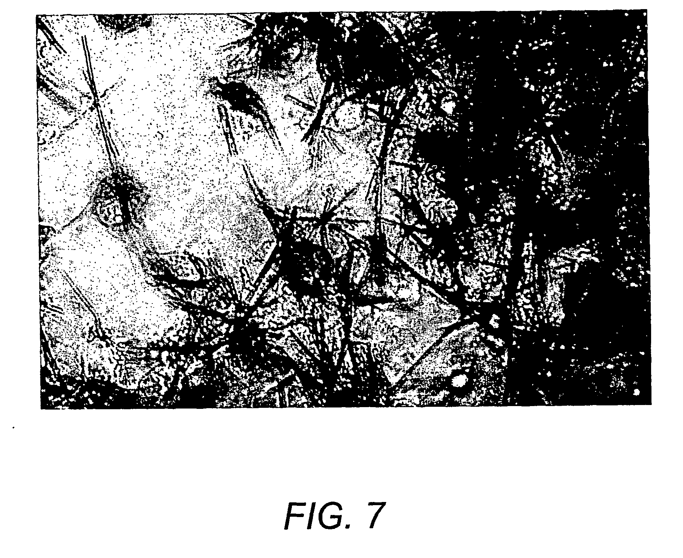

9L gliosarcoma cancer cells were exposed to TR (50 ug/ml), a substrate of SDH. The tumor cells (4000) were seeded one day before being treated with TR. As can be seen in Figure 7, nuclear envelopes and/or endoplasmic reticulum in most cells were frozen by the crystals formed on them.

## Claims

1. A tetrazolium salt for use in the treatment of cancer, wherein said salt as a cellular organelle crystallizing agent is suitable for forming visible crystals in an organelle of a cell through a reaction catalyzed by cellular enzymes.

2. A tetrazolium salt for use according to claim 1, selected from one of the group consisting of p-Anisyl Blue Tetrazolium Chloride, p-Anisyl-p-Nitro Blue Tetrazolium Chloride, Thiazolyl blue (2-2'-Benzothiazolyl-5-styryl-3- (4'-phthalhydrazidyl) tetrazolium chloride), Blue tetrazolium chloride, 2-(2'-Bemothiazolyl)-5-styryl-3- (4'-phthalhydrazidyl)-tetrazolium chloride, 5-Cyano-2,3- ditolyl tetrazolium chloride), [3- (4, 5-Dimethylthiazol-2-yl)-2,5-diphenyl] tetrazolium Bromide, 1- [4, 5-dimethylthiazol-2-yl]-2, 5-diphenyltetrazolium bromide, Distyryl nitroblue tetrazolium chloride, (1H)-tetrazole, Iodonitrotetrazolium chloride, Iodo Nitro Tetrazolium Violet Chloride, p-iodo nitrotetrazolium violet, (2- (4-iodophenyl)-3-(4- nitrophenyl)-5-phenyltetrazolium chloride, 2-(p-iodophenyl)-p-nitrophenyl-5-phenyltetrazolium chloride, m-Nitro Blue Tetrazolium Chloride, m-Nitro Neotetrazolium Chloride, 2,2-bis(2-methoxyl-4-nitro-5-sulfophenyl)-2H-tetazolium-5-carboxanilide, 3-(4,5-dimethylthiazol-2-yl)-5-(3-carboxymethoxyphenyl)-2-(4-sulfophenyl)-2H- tetrazolium salt, tetrazolium bromide, thiazolyl blue tetrazolium bromide, 3→4, 5-dimethylthiazol-2-yl-2,5-diphenyltetrazolium bromide, Nitro Blue Monotetrazolium Chloride, p-Nitro Blue Tetrazolium Chloride, Nitro blue tetrazolium chloride, 2,2'-dinitrophenyl-5,5'-diphenyl-3, 3'-(3, 3'-dimethoxy-4,4'-diphenylene) ditetrazolium chloride, Neotetrazolium chloride, 2,2',5,5'-Tetraphenyl-3,3'-(p-diphenylene) ditetrazolium chloride, Nitrotetrazolium Violet; Thiazolyl blue, tetrazolium blue chloride, 3,3'→3,3'- dimethoxy (1, 1'-biphenyl)-4,4'-diyl]-bis(2,5-diphenyl-2H-tetrazolium) dichloride, Nitroblue tetrazolium chloride, o-Tolyl Tetrazolium, Red, sodium 3'-[1-[(phenylamino)-carbonyl]-3,4-tetrazolium]-bis(4-methoxy-6-nitro) benzene-sulfonic acid hydrate, p-Nitro Blue Tetrazolium Chloride, Piperonyl Tetrazolium Blue, p-Tolyl Tetrazolium Red, Thiocarbamyl nitro blue tetrazolium chloride, 2,2'-di-p-nitrophenyl-5,5'-di-p- thiocarbamylphenyl-3,3' [3,3'-dimethoxy-4,4'-biphenylene]-ditetrazolium chloride, Tetranitroblue tetrazolium chloride, 1,3,5-triphenyltetrazolium, Tetrazolium Red, 2,3,5- triphenyltetrazolium chloride), Tetrazolium violet, Violet Tetrazolium, 2,3,5-Triphenyl-2-H-tetrazolium chloride, 2,5-diphenyl-3-[alpha-naphthyl]-tetrazolium chloride, 2,5- diphenyl-3- [1-naphthyl]-2H-tetrazolium chloride, Veratryl tetrazolium blue, 4-[3-(4- iodophenyl)-2-(4-nitrophenyl)-2H-5-tertrazolio]-1, 3-benzene disulfonate, 2,2-bis (2- methoxyl-4-nitro-5-sulfophenyl)-2H-tetrazolium-5-carboxanilide.

3. A tetrazolium salt for use according to claim 1, selected from the group consisting of tetrazolium violet and MTT.

4. A tetrazolium salt for use according to claim 1, wherein the salt is administered in an effective amount in the range 0.001 µg/kg/day to 1000 mg/kg/day.

5. A tetrazolium salt for use according to claim 1, wherein the salt is used with non-surgical therapy selected from the group consisting of chemotherapy, radiation therapy, hyperthermia therapy, hormone therapy, immunotherapy, photodynamic therapy, electric therapy, gene therapy, ultrasound, laser, hyperbaric oxygen therapy, antiangiogenesis therapy, and ischemic-reperfusion therapy.

6. A tetrazolium salt for use according to claim 5, wherein the gene therapy increases the activity and/or expression of dehydrogenases.

7. A tetrazolium salt according to claim 1 and a pharmaceutically acceptable carrier, for use in the treatment of cancer.

8. A tetrazolium salt for use according to claim 1, wherein the cancer is selected from the group consisting of carcinoma, sarcoma, carcinosarcoma, lymphoma, and leukemia.

## Patentansprüche

1. Tetrazoliumsalz zur Verwendung bei der Behandlung von Krebs, wobei das genannte Salz als ein Zellorganellen-Kristallisierungsmittel zur Bildung sichtbarer Kristalle in einer Organelle einer Zelle über eine durch zelluläre Enzyme katalysierte Reaktion geeignet ist.

2. Tetrazoliumsalz zur Verwendung gemäß Anspruch 1, das ausgewählt ist aus der Gruppe bestehend aus p-Anisylblau-Tetrazoliumchlorid, p-Anisyl-p-Nitroblau-Tetrazoliumchlorid, Thiazolylblau (2-2'-Benzothiazolyl-5-styryl-3-(4'-phthalhydrazidyl)tetrazoliumchlorid), slautetrazoliumchlorid, 2-(2'-Benzothiazolyl)-5-styryl-3-(4'-phthalhydrazidyl)-tetrazoliumchlorid, 5-Cyano-2,3-ditolyl-tetrazoliumchlorid), [3-(4,5-Dimethylthiazol-2-yl)-2,5-diphenyl]tetrazoliumbromid, 1-[4,5-Dimethylthiazol-2-yl]-2,5-diphenyltetrazoliumbromid, Distyryl-Nitroblau-Tetrazoliumchlorid, (1H)-Tetrazol, Iodnitrotetrazoliumchlorid, Iodnitrotetrazoliumviolettchlorid, p-Iodnitrotetrazoliumviolett, (2-(4-Iodphenyl)-3-(4-nitrophenyl)-5-phenyltetrazoliumchlorid, 2-(p-Iodphenyl)-p-nitrophenyl-5-phenyltetrazoliumchlorid, m-Nitroblau-Tetrazoliumchlorid, m-Nitro-Neotetrazoliumchlorid, 2,2-bis(2-Methoxyl-4-nitro-5-sulfophenyl)-2H-tetrazolium-5-carboxanilid, 3-(4,5-Dimethylthiazol-2-yl)-5-(3-carboxymethoxyphenyl)-2-(4-sulfophenyl)-2H-tetrazoliumsalz, Tetrazoliumbromid, Thiazolylblau-Tetrazoliumbromid, 3-4,5-Dimethylthiazol-2-yl-2,5-diphenyltetrazoliumbromid, Nitroblaumonotetrazoliumchlorid, p-Nitroblau-Tetrazoliumchlorid, Nitroblau-Tetrazoliumchlorid, 2,2'-Dinitrophenyl-5,5'-diphenyl-3,3'-(3,3'-dimethoxy-4,4'-diphenylen)ditetrazoliumchlorid, Neotetrazoliumchlorid, 2,2',5,5'-Tetraphenyl-3,3'-(p-diphenylen)ditetrazoliumchlorid, Nitrotetrazoliumviolett; Thiazolylblau, Tetrazoliumblauchlorid, 3,3'→3,3'-Dimethoxy(1,1'-biphenyl)-4,4'-diyl]-bis(2,5-diphenyl-2H-tetrazolium)dichlorid, Nitroblau-Tetrazoliumchlorid, o-Tolyltetrazoliumrot, Natrium-3'-[1-[(phenylamino)-carbonyl]3,4-tetrazolium]-bis(4-methoxy-6-nitro)benzolsulfonsäurehydrat, p-Nitroblau-Tetrazoliumchlorid, Piperonyltetrazoliumblau, p-Tolyltetrazoliumrot, Thiocarbamylnitroblau-Tetrazoliumchlorid, 2,2'-Di-p-nitrophenyl-5,5'-di-p-thiocarbamylphenyl-3,3'[3,3'-dimethoxy-4,4'-biphenylen]-ditetrazoliumchlorid, Tetranitroblau-Tetrazoliumchlorid, 1,3,5-Triphenyltetrazolium, Tetrazoliumrot, 2,3,5-Triphenyltetrazoliumchlvrid), Tetrazoliumviolett, Violetttetrazolium, 2,3,5-Triphenyl-2-H-tetrazoliumchlorid, 2,5-Diphenyl-3-[alpha-naphthyl]-tetrazoliumchlorid, 2,5-Diphenyl-3-[1-naphthyl]-2H-tetrazoliumchlorid, Veratryltetrazoliumblau, 4-[3-(4-Iodphenyl)-2-(4-nitrophenyl)-2H-5-tetrazolio]-1,3-benzoldisulfonat, 2,2-bis(2-Methoxyl-4-nitro-sulfophenyl)-2H-tetrazolium-5-carboxanilid.

3. Tetrazoliumsalz zur Verwendung gemäß Anspruch 1, das ausgewählt ist aus der Gruppe bestehend aus Tetrazoliumviolett und MTT.

4. Tetrazoliumsalz zur Verwendung gemäß Anspruch 1, wobei das Salz in einer effektiven Menge im Bereich von 0,001 µg/kg/Tag bis 1000 mg/kg/Tag verabreicht wird.

5. Tetrazoliumsalz zur Verwendung gemäß Anspruch 1, wobei das Salz bei einer nicht-operativen Therapie verwendet wird, die ausgewählt ist aus der Gruppe bestehend aus Chemotherapie, Strahlentherapie, Hyperthermietherapie, Hormontherapie, Immuntherapie, photodynamischer Therapie, Elektrotherapie, Gentherapie, Ultraschall, Laser, hyperbarer Sauerstofftherapie, Antiangiogenesetherapie und Ischämie-/Reperfusionstherapie,

6. Tetrazoliumsalz zur Verwendung gemäß Anspruch 5, wobei die Gentherapie die Aktivität und/oder Expression von Dehydrogenasen erhöht.

7. Tetrazoliumsalz nach Anspruch 1 und ein pharmazeutisch akzeptabler Träger zur Verwendung bei der Behandlung von Krebs.

8. Tetrazoliumsalz zur Verwendung gemäß Anspruch 1, wobei der Krebs ausgewählt ist aus der Gruppe bestehend aus Karzinom, Sarkom, Karzinosarkom, Lymphom und Leukämie.

## Revendications

1. Sel de tétrazolium à utiliser dans le traitement du cancer, ledit sel, en tant qu'agent de cristallisation des organites cellulaires, étant adéquat pour former des cristaux visibles dans un organite d'une cellule par l'intermédiaire d'une réaction catalysée par des enzymes cellulaires.

2. Sel de tétrazolium à utiliser selon la revendication 1, ledit sel étant sélectionné parmi le groupe constitué par : p-anisyl bleu de tétrazolium chlorure, p-anisyl-p-bleu nitré de tétrazolium chlorure, bleu de thiazolyl (2-2'-benzothiazolyl-5-styryl-3-(4'-phtalhydrazidyl) tétrazolium chlorure), bleu de tétrazolium chlorure, 2-(2'-benzothiazolyl)-5-styryl-3-(4'-phtalhydrazidyl)-tétrazolium chlorure, 5-cyano-2,3-ditolyl tétrazolium chlorure), [3-(4,5-diméthylthiazol-2-yl)-2,5-diphényl] tétrazolium bromure, 1-[4,5-diméthylthiazol-2-yl]-2,5-diphényltétrazolium bromure, distyryl-bleu nitré de tétrazolium chlorure, (1H)-tétrazole, iodonitrotétrazolium chlorure, iodonitro-violet de tétrazolium chlorure, p-iodonitro-violet de tétrazolium (2-(4-iodophényl)-3-(4-nitrophényl)-5-phényltétrazolium chlorure, 2-(p-iodophényl)-p-nitrophényl-5-phényltétrazolium chlorure, m-bleu nitré de tétrazolium chlorure, m-nitro-néotétrazolium chlorure, 2,2-bis(2-méthoxyl-4-nitro-5-sulfophényl)-2H-tétrazolium-5-carboxanilide, 3-(4,5-diméthylthiazol-2-yl)-5-(3-carboxyméthoxyphényl)-2-(4-sulfophényl)-2H-sel de tétrazolium, tétrazolium bromure, thiazolyl bleu de tétrazolium bromure, 3→4,5-diméthylthiazol-2-yl-2,5-diphényltétrazolium bromure, bleu nitré de monorétrazolium chlorure, p-bleu nitré de tétrazolium chlorure, bleu nitré de tétrazolium cholure, 2,2'-di-nitrophényl-5,5'-diphényl-3, 3'-(3,3'-diméthoxy-4,4'-diphénylène) ditétrazolium chlorure, néotétrazolium chlorure, 2,2',5,5'-tétraphényl-3,3'-(p-diphénylène) ditétrazolium chlorure, violet nitré de tétrazolium ; bleu de thiazolyl, bleu de tétrazolium chlorure, 3.3'→3,3'-diméthoxy(1,1'-biphényl)-4,4'-diyl]-bis(2,5-diphcnyl-2H-tétrazolium) dichlorure, bleu nitré de tétrazolium chlorure, o-tolyl rouge de tétrazolium, sodium 3'-[1-[(phénylamino)-carbonyl]-3,4-tétrazolium]-bis(4-méthoxy-6-nitro) benzène-hydrate d'acide sulfonique, p-bleu nitré de tétrazolium chlorure, pipéronyl bleu de tétrazolium, p-lolyl rouge de tétrazolium, thiocarbamyl bleu nitré de tétrazolium chlorure, 2,2'-di-p-nitrophényl-5,5'-di-p-thiocarbamylphényl-3,3' [3,3'-diméthoxy-4,4'-biphénylène]-ditétrazolium chlorure, tétra-bleu nitré de tétrazolium chlorure, 1,3,5-triphényltétrazolium, rouge de tétrazolium, 2,3,5-triphényltétrazolium chlorure), violet de tétrazolium, violet tétrazolium, 2,3,5-triphényl-2-H-tétrazolium chlorure, 2,5-diphényl-3-[alpha-naphtyl]-tétrazolium chlorure, 2,5-diphényl-3-[1-naphtyl]-2H-tétrazolium chlorure, vératryl bleu de tétrazolium, 4-[3-(4-iodophényl)-2-(4-nitrophényl)-2H-5-tétrazolio)-1, 3-benzène disulfonate, 2,2-bis (2-méthoxyl-4-nitro-5-sulfophényl)-2H-tétrazolium-5-carboxanilide.

3. Sel de tétrazolium à utiliser selon la revendication 1, ledit sel étant sélectionné parmi le groupe constitué par le violet de tétrazolium et le MTT.

4. Sel de tetrazolium à utiliser selon la revendication 1, ledit sel étant administré en une quantité efficace comprise entre 0,001 µg/kg/jour et 1000 mg/kg/jour.

5. Sel de tétrazolium à utiliser selon la revendication 1, ledit sel étant utilisé avec une thérapie non chirurgicale sélectionnée parmi le groupe constitué par la chimiothérapie, la radiothérapie, la thérapie par hyperthermie, l'hormonothérapie, l'immunothérapie, la thérapie photodynamique, l'électrothérapie, la thérapie génique, les ultrasons, le laser, l'oxygénothérapie hyperbare, la thérapie antiangiogénique et la thérapie d'ischémie-reperfusion.

6. Sel de tétrazolium à utiliser selon la revendication 5, la thérapie génique augmentant l'activité et/ou l'expression des déshydrogénases.

7. Sel de tétrazolium selon la revendication 1 et un vecteur pharmaceutiquement acceptable, à utiliser dans le traitement du cancer.

8. Sel de tétrazolium à utiliser selon la revendication 1, le cancer étant sélectionné parmi le groupe constitué par le carcinome, le sarcome, le carcinosarcome, le lymphome et la leucémie.
